# EUROPEAN PATENT APPLICATION

(11) **EP 1 710 310 A1**
(43) Date of publication of application: **11.10.2006**
(21) Application number: 04807986.7
(22) Date of filing: 28.12.2004
(51) Int. Cl.: C12N 15/31, C12N 1/20, C12Q 1/68, C12Q 1/02, C12P 21/08, C07K 16/12, G01N 33/53, G01N 33/569, C12M 1/00

(54) **NOVEL SERUM TYPE STREPTOCOCCUS MUTANS AND UTILIZATION OF THE SAME**

(30) Priority: 30.12.2003 US 533076 P; 31.03.2004 JP 2004106825
(71) Applicant: SUNTORY LIMITED, Osaka-shi, Osaka 530-8203 (JP)
(72) Inventor: OOSHIMA, Takashi, Osaka 565-0816 (JP); NAKANO, Kazuhiko, Osaka 567-0821 (JP)
(74) Representative: Vossius & Partner
(86) International application number: PCT/JP2004/019631
(87) International publication number: WO 2005/063992

(57) **Abstract**

A polynucleotide encoding a polypeptide which lowers the glucose side chain amount in a serotype-specific polysaccharide antigen of *Streptococcus mutans*; novel *Streptococcus mutans* strain having the above polynucleotide; an antibody specific to this *Streptococcus mutans* strain; and a method of detecting the above-described *Streptococcus mutans* strain occurring in a subject sample. According to this method, it is possible to examine the presence or absence of *Streptococcus mutans* of an untypable serotype in a subject sample to thereby specify a subject having a high risk of the onset of infective endocarditis.

## Description

### FIELD OF THE INVENTION

The present invention relates to serologically novel strains of *Streptococcus mutans (S. mutans);* antibodies specific to the novel strains; a method for producing the antibodies; a method and kit for detecting the novel strains with the antibodies; polynucleotides that encode enzymes associated with the biosynthesis of a polysaccharide antigen specific to the novel strains of *S. mutans*; and a method and kit for detecting the novel strains with the polynucleotides. Specifically, the invention relates to a method for detecting the presence of novel strains of *S. mutans* in subjects, and a kit for performing the method, either by using the antibodies in an immunization reaction with surface polysaccharide antigens extracted from subject samples, or by using the polynucleotides in a hybridization reaction with the genomic DNA extracted from subject samples, or in a PCR reaction.

### BACKGROUND OF THE INVENTION

*S. mutans* is known to be a major causative bacterium of dental caries in humans. Dental caries is a disorder in which the teeth are dissolved by acids generated by *S. mutans. S. mutans* proliferates in the oral cavity and secretes the enzyme, glucosyltransferase. The enzyme decomposes sugars in foods, and generates water-insoluble polysaccharide glucan. The glucan combines with the bacteria *S. mutans* and forms plaque (dental plaque) that adheres to the surface of the teeth. In the plaque, *S. mutans* metabolizes the sugar and generates acids such as lactic acid. The acid dissolves calcium from enamel of the surface of the teeth, resulting in dental caries. Dental caries, therefore, is a serious disorder in the field of dentistry, and there have been ongoing studies on treatment and prevention of dental caries.

Known methods of bacterial classification include: a method based on physiobiochemical properties; a method based on compositions of quinine, bacterial fatty acid, or cell wall; a method based on G+C content of DNA; a method based on DNA homology; a method using DNA probes; and a method that analyzes the base sequence of 16S ribosomal RNA (rRNA) gene. In the classification and identification of bacteria that are commonly carried out these days, phylogenetic groups (clusters) that are classified based on the base sequence of 16S ribosomal RNA gene are used as an index.

Based on their differences in composition and the linkage of cell wall polysaccharides, mutans streptococci are classified into 8 serotypes as follows; *Streptococcus mutans* (serotype c, e, f), *Streptococcus* sorbinus (d, g), *Streptococcus cricetus* (a), *Streptococcus rattus* (b), *Streptococcus ferus* (c), *Streptococcus macacae* (c), and *Streptococcus downei* (h).

*S. mutans* is occasionally isolated from the blood of patients with infective endocarditis (IE). IE is one of the most common systemic disorders known in dentistry. IE is a fatal infectious disease which is often seen in patients who have undergone replacement of the heart valves or patients with congenital malformation. IE is caused upon entry of causative bacteria into the blood of patients during dental care, whereby an inflammation is caused by the bacteria establishing hold on the endocardium. The causative bacteria are usually streptococci (commonly, *Streptococcus sanguinis*) that are present in the oral cavity. Among other, *S. mutans* also classifies as one of the causative bacteria.

In dentistry, patients with IE are commonly treated by first administering antibiotics prior to the actual treatment, in which the procedure is completed before the raised concentration level of the antibiotics falls (see Non-Patent Documents 1 and 2).

### [Non-Patent Document 1]

Dajani A. S. et al., Prevention of bacterial endocaridtis; Recommendations by the American Heart Association. Circulation 1997 96: 358-366.

### [Non-Patent Document 2]

Nakatani S et al., Current characteristics of infective endocarditis in Japan ; an analysis of 848 cases in 2000 and 2001. Circ. J. 2003 67: 901-905.

The antibiotics are used in large amounts for the prevention of IE. For example, a penicillin antibiotic, amoxicillin, is preoperatively administered to a 25 kg child in 1000 mg each dose, either in the form of a capsule or a powder.

While the use of such a large amount of antibiotic is medically necessary, the problem of drug-resistant bacteria that arise from overuse of antibiotics has become a serious issue. Thus, it would be useful to reduce the amount of antibiotic and suppress the emergence of drug-resistant bacteria due to overuse of antibiotics.

In a previous study of the inventors, 4 streptococcal strains isolated from patients with IE or bacteremia following a tooth extraction procedure were specified as *S. mutans* based on their biological properties and 16S ribosomal RNA alignment (see Non-Patent Document 3). Generally, about 80% of *S. mutans* strains are classified as serotype c. However, none of the 4 strains of *S. mutans* isolated from the patient blood was serotype c. Two of the strains (TW295 and TW871) were shown to be serologically untypable, neither belonging to any of the known serotypes *c*, *e*, and *f*, while the others belonged to serotype *e* or *f*.

The serotype specific polysaccharide antigens that determine the serotype of *S. mutans* are composed of rhamnose-glucose polymers, with a backbone of rhamnose and side chains of α- or β-linked glucosidic residues. The serologically untypable properties in strains of TW295 and TW871 have been shown to be derived from the lack of a glucose side chain in the serotype specific polysaccharide. Further, the serotype-specific polysaccharide antigens of these serologically untypable strains (TW295 and TW871) (conveniently, "serologically untypable strains of *S. mutans")* share similarities with those of the mutant strain with inactivation of *gluA* gene, which encodes the enzyme that catalyzes the production of the immediate precursor of the glucose side chain donor, in that they all had low glucose contents.

The serologically untypable isolate strains were shown to have hydrophobicity and sucrose-dependent adhesion at levels as high as the oral isolates, and were less susceptible to phagocytosis.

### [Non-Patent Document 3]

Fujiwara, T., K. Nakano, M. Kawaguchi, T. Ooshima, S. Sobue, S. Kawabata, I. Nakagawa, and S. Hamada. 2001. Biochemical and genetic characterization of serologically untypable Streptococcus mutans strains isolated from patients with bacteremia. Eur. J. Oral Sci. 109: 330-334.

These findings imply that the serologically untypable strains of *S. mutans* may be present in the oral cavity of humans, because of their high cellular hydrophobicity and sucrose-dependent adherence levels. Further, they may be able to survive in blood longer owing to their low susceptibility to phagocytosis during invasion of blood. This may account for the isolation of these strains from the blood.

For the prevention of IE, it is highly desirable to conveniently identify patients carrying causative bacteria (for example, serologically untypable strains of *S. mutans*) of the disease. Further, specifying causative bacteria of IE would be useful for the selection of antibiotics to be used in treatment. However, the development of these methods is still under way.

As described above, the serologically untypable strains of *S. mutans* remain stable in blood. This might account for the high onset rate of IE in patients infected with these strains. The serologically untypable strains of *S. mutans* are therefore considered to be important in the prevention of potentially fatal IE. Accordingly, there is a need for isolation and identification of serologically untypable strains of *S. mutans.*

An object of the present invention is to provide a method by which serologically untypable strains of *S. mutans* are isolated and identified, so as to effectively and efficiently determine whether the untypable strains are present in a subject.

### SUMMARY OF THE INVENTION

The inventors of the present invention diligently worked to solve the foregoing problems, and obtained antisera to serologically untypable *S. mutans* strains by making modifications to conventional immunization methods. These *S. mutans* strains were designated as new serotype *k*.

In order to analyze the polysaccharide antigen that determines the new serotype *k* strains, the entire sequences of genes that encode enzymes *(rgpA, rgpB, rgpC, rgpD, rgpE, rgpF,* ORF7, *rgpH, rgpI,* and ORF10) associated with the biosynthesis of *S. mutans* polysaccharide antigen were determined in the serotype *k* strains. Comparisons of gene sequences between serotype *k* strains and *S. mutans* strains of known serotypes revealed that a specific sequence was commonly present in *rgpF* of different serotype *k* strains. Further, primers that were designed based on the specific base sequence in *rgpF* of serotype *k* strains were used for the PCR amplification of tissue samples obtained from subjects. This was found to be effective in efficiently detecting the presence of untypable strains of *S. mutans* in the subjects. The present invention was made based on these findings.

Specifically, a polynucleotide, or a fragment thereof, of the present invention includes a base sequence, or a complementary sequence thereof, that is specific to *Streptococcus mutans* strains with a reduced amount of glucose side chain of the serotype-specific polysaccharide antigen of *S. mutans.*

Preferably, a polynucleotide, or a fragment thereof, of the present invention is a polynucleotide that encodes a polypeptide that reduces the amount of glucose side chain of the polysaccharide antigen specific to *S. mutans.*

It is preferable that the polynucleotide comprise:
a base sequence of any of SEQ ID NO: 1 through 4; or
a base sequence with the deletion, substitution, or addition of one or more bases in the base sequence of any of SEQ ID NO: 1 through 4.

It is preferable that the polynucleotide comprise:
a polynucleotide of a base sequence of any of SEQ ID NO: 1 through 4; or
a polynucleotide that hybridizes under stringent conditions with a polynucleotide having a complementary base sequence to the polynucleotide of the base sequence of any of SEQ ID NO: 1 through 4.

It is more preferable that the polynucleotide comprise a base sequence of any of SEQ ID NO: 1 through 4.

According to the foregoing construction, there is provided a polynucleotide that is present specific to *Streptococcus mutans* strains of a new serotype.

Specifically, an oligonucleotide of the present invention includes a base sequence, or a complementary sequence thereof, that is specific to *Streptococcus mutans* strains with a reduced amount of glucose side chain of the serotype specific polysaccharide antigen of *S. mutans.*

Preferably, the oligonucleotide comprises a base sequence, or a complementary sequence thereof, with at least 12 contiguous bases of a base sequence of any of SEQ ID NO: 1 through 4.

Preferably, the oligonucleotide comprises a base sequence, or a complementary sequence thereof, of any of SEQ ID NO: 8 through 10.

According to the foregoing construction, there is provided an oligonucleotide that excels in specifically detecting *Streptococcus mutans* strains of a new serotype.

More specifically, a polypeptide of the present invention reduces the amount of glucose side chain of the polysaccharide antigen specific to *S. mutans.*

Preferably, a polypeptide of the present invention is encoded by the polynucleotide, or a fragment thereof, as defined above.

More preferably, a polypeptide of the present invention is encoded by a polynucleotide of a base sequence of any of SEQ ID NO: 1 through 4.

According to the foregoing construction, there is provided a polypeptide that is present specific to *Streptococcus mutans* strains of a new serotype other than *c, e,* or, *f* strains.

More specifically, *Streptococcus mutans* strains of the present invention have a reduced amount of glucose side chain in the polysaccharide antigen specific to *S. mutans.*

Preferably, *Streptococcus mutans* strains of the present invention are of a new serotype with a reduced amount of glucose side chain in the serotype specific polysaccharide antigen of *S. mutans.*

In one aspect of the invention, *Streptococcus mutans* strains of the present invention has preferably the polynucleotide as defined above.

In another aspect, *Streptococcus mutans* strains of the present invention preferably express the polypeptide as defined above.

According to the foregoing construction, there is provided new information concerning *S. mutans* of known serotypes, and there is also provided *Streptococcus mutans* strains that are useful in the treatment and/or prevention of diseases which could not be solved by *S. mutans* strains of known serotypes.

More specifically, an antibody of the present invention specifically binds to the polysaccharide antigen of *S. mutans* strains that have a reduced amount of glucose side chain in the polysaccharide antigen specific to *c, e,* or *f* strains of *S. mutans.*

In other words, an antibody of the present invention binds to the polysaccharide antigen that is specific to *Streptococcus mutans* strains in which the amount of glucose side chain of the serotype-specific polysaccharide antigen is reduced.

Preferably, an antibody of the present invention specifically binds to the serotype-specific polysaccharide antigen of *S. mutans* strains.

According to the foregoing construction, there is provided an antibody useful for the detection of *Streptococcus mutans* strains that have a reduced amount of glucose side chain in the serotype-specific polysaccharide antigen of *S. mutans,* i.e., *S. mutans* strains of a new serotype.

More specifically, a method for detecting *S. mutans* strains in a subject sample according to the present invention includes the step of carrying out a PCR reaction using the oligonucleotide as a primer.

Preferably, a method for detecting *S. mutans* strains in a subject sample according to the present invention further includes the step of separating bacteria from the subject sample, and the step of extracting genomic DNA or total RNA of the separated bacteria.

In a method for detecting *S. mutans* strains in a subject sample according to the present invention, it is preferable that the tissue sample be obtained from blood, saliva, or plaque.

In a method for detecting *S. mutans* strains in a subject sample according to the present invention, it is preferable that the primers are an oligonucleotide comprising a base sequence of SEQ ID NO: 8, and an oligonucleotide comprising a base sequence of SEQ ID NO: 9 or 10.

According to the foregoing construction, the presence of new serotype strains can be found by detecting genes that encode enzymes important for the biosynthesis of the serotype-specific polysaccharide antigen of the *S. mutans* new serotype strains.

More specifically, in order to solve the foregoing problems, a method for detecting *S. mutans* strains in a subject sample according to the present invention includes the step of carrying out a hybridization reaction using the oligonucleotide as a probe.

Preferably, a method for detecting *S. mutans* strains in a subject sample according to the present invention includes the step of separating bacteria from a subject sample, and the step of extracting genomic DNA or total RNA of the separated bacteria.

In a method for detecting *S. mutans* strains in a subject sample according to the present invention, it is preferable that the tissue sample be obtained from blood, saliva, or plaque.

In a method for detecting *S. mutans* strains in a subject sample according to the present invention, it is preferable that the oligonucleotide comprise a base sequence of any of SEQ ID NO: 8 through 10.

In a method for detecting *S. mutans* strains in a subject sample according to the present invention, it is preferable that the antibody be used in the step of separating bacteria.

More specifically, in order to solve the foregoing problems, a method for determining a serotype of *S. mutans* strains by a hybridization reaction according to the present invention is adapted so that a probe used in the hybridization reaction is an oligonucleotide that includes a base sequence, or a complementary sequence thereof, that is specific to the *S. mutans* strains in which the amount of glucose side chain of the serotype-specific polysaccharide antigen of *S. mutans* is reduced, and that the specific base sequence includes at least 12 contiguous bases of the base sequence of any of SEQ ID NO: 1 through 4.

Preferably, a method for determining a serotype of *S. mutans* strains by a hybridization reaction according to the present invention is adapted so that a probe used in the hybridization reaction is an oligonucleotide that includes a base sequence, or a complementary sequence thereof, that includes at least 12 contiguous bases of the base sequence specific to serotype *c, e,* or *f S. mutans* strains.

Preferably, a method for determining a serotype of *S. mutans* strains by a hybridization reaction according to the present invention further includes the step of obtaining chromosomal DNA or total RNA from the tissue sample.

Preferably, a method for determining a serotype of *S. mutans* strains by a hybridization reaction according to the present invention is adapted so that the tissue sample is obtained from blood, saliva, or plaque.

Preferably, a method for determining a serotype of *S. mutans* strains by a hybridization reaction according to the present invention further includes the step of separating bacteria from saliva or plaque.

According to the foregoing construction, there is provided a method for determining a serotype of *S. mutans* strains, whereby the method excels in specifically detecting *S. mutans* strains in which the amount of glucose side chain of polysaccharides specific to serotype *c, e,* or *f* strains of *S. mutans* is reduced.

According to the foregoing construction, the presence of new serotype strains can be found by detecting enzymes that are important for the biosynthesis of the serotype-specific polysaccharide antigen of *S. mutans* new serotype strains.

More specifically, in order to solve the foregoing problems, a method for detecting *S. mutans* strains that have a reduced amount of glucose side chain in the serotype-specific polysaccharide antigen of *S. mutans* according to the present invention includes the step of carrying out an immunization reaction with the antibody.

It is preferable that a method for detecting *S. mutans* strains in a subject sample according to the present invention include the steps of:
separating bacteria from the subject sample;
incubating the separated bacteria with an antibody; and
detecting bacteria that have bound to the antibody.

According to the foregoing construction, there is provided a method useful for the detection of *S. mutans* of a new serotype.

More specifically, a method for determining a serotype of *S. mutans* strains in a subject sample according to the present invention uses the method of detecting *S. mutans* strains.

More specifically, in order to solve the foregoing problems, a method for determining a serotype of *S. mutans* strains by a PCR reaction according to the present invention is adapted so that a primer used in the PCR reaction is an oligonucleotide that includes a base sequence, or a complementary sequence thereof, that is specific to the *S. mutans* strains in which the amount of glucose side chain of the serotype-specific polysaccharide antigen of *S. mutans* is reduced, and that the specific base sequence includes at least 12 contiguous bases of the base sequence of any of SEQ ID NO: 1 through 4.

Preferably, a method for determining a serotype of *S. mutans* strains by a PCR reaction according to the present invention is adapted so that a primer used in the hybridization reaction is an oligonucleotide that includes a base sequence, or a complementary sequence thereof, that includes at least 12 contiguous bases of the base sequence specific to serotype *c, e,* or *f S. mutans* strains.

Preferably, a method for determining a serotype of *S. mutans* strains by a PCR reaction according to the present invention further includes the step of obtaining chromosomal DNA or total RNA from the tissue sample of a subject.

Preferably, a method for determining a serotype of *S. mutans* strains by a PCR reaction according to the present invention is adapted so that the tissue sample is obtained from blood, saliva, or plaque.

Preferably, a method for determining a serotype of *S. mutans* strains by a PCR reaction according to the present invention further includes the step of separating bacteria from saliva or plaque.

According to the foregoing construction, there is provided a method for determining a serotype of *S. mutans* strains, whereby the method excels in specifically detecting *S. mutans* strains in which the amount of glucose side chain of the serotype-specific polysaccharide antigen of *S. mutans* is reduced.

More specifically, in order to solve the foregoing problems, a method for determining a serotype of *S. mutans* strains uses an antibody that specifically binds to *S. mutans* strains in which the amount of glucose side chain of the serotype-specific polysaccharide antigen of *S. mutans* is reduced.

Preferably, a method for determining a serotype of *S. mutans* strains uses an antibody that specifically binds to *S. mutans* strains in which the amount of glucose side chain of the serotype-specific polysaccharide antigen of *S. mutans* is reduced.

According to the foregoing construction, there is provided a method for determining a serotype of *S. mutans* strains, whereby the method excels in specifically detecting *S. mutans* strains in which the amount of glucose side chain of the serotype-specific polysaccharide antigen of *S. mutans* is reduced.

More specifically, a screening method of *S. mutans* strains of the present invention uses the method of detecting *S. mutans* strains.

More specifically, a screening method of *S. mutans* strains in which the amount of glucose side chain of the serotype-specific polysaccharide antigen of *S. mutans* is reduced according to the present invention uses the polynucleotide, or a fragment thereof, as defined above.

Preferably, a screening method of *S. mutans* strains in which the amount of glucose side chain of the serotype-specific polysaccharide antigen of *S. mutans* is reduced according to the present invention is adapted so that the polynucleotide, or a fragment thereof, is used for the PCR reaction.

Preferably, a screening method of *S. mutans* strains in which the amount of glucose side chain of the serotype-specific polysaccharide antigen of *S. mutans* is reduced according to the present invention is adapted so that the polynucleotide, or a fragment thereof, is used for the hybridization reaction.

Preferably, a screening method of *S. mutans* strains in which the amount of glucose side chain of the serotype-specific polysaccharide antigen of *S. mutans* is reduced according to the present invention uses the antibody as defined above.

According to the foregoing construction, there is provided a screening method, whereby the method excels in screening for *S. mutans* strains in which the amount of glucose side chain of the serotype-specific polysaccharide antigen of *S. mutans* is reduced.

More specifically, *S. mutans* strains of the present invention are obtained by the screening method as defined above.

More specifically, *S. mutans* strains of the invention in which the amount of glucose side chain of the serotype-specific polysaccharide antigen of *S. mutans* is reduced are separated by the screening method as defined above.

According to the foregoing construction, there are provided newly screened *S. mutans* strains in which the amount of glucose side chain of the serotype-specific polysaccharide antigen of *S. mutans* is reduced.

More specifically, in order to solve the foregoing problems, *S. mutans* strains of the invention in which the amount of glucose side chain of the serotype-specific polysaccharide antigen of *S. mutans* is reduced are screened by the screening method as defined above.

According to the foregoing construction, there are provided *S. mutans* strains of a new serotype.

More specifically, a kit for detecting *S. mutans* strains of the present invention includes the oligonucleotide as defined above.

In a kit for detecting *S. mutans* strains of the present invention, the oligonucleotide is preferably an oligonucleotide of a base sequence of SEQ ID NO: 9.

It is preferable that a kit for detecting *S. mutans* strains of the present invention include an oligonucleotide of a base sequence of SEQ ID NO: 8.

It is preferable that a kit for detecting *S. mutans* strains of the present invention include an oligonucleotide of a base sequence of SEQ ID NO: 10.

It is preferable that a kit for detecting *S. mutans* strains of the present invention be used for a PCR reaction or a hybridization reaction.

More specifically, in order to solve the foregoing problems, a kit for determining a serotype of *S. mutans* strains of the present invention includes an oligonucleotide that includes a base sequence, or a complementary sequence thereof, that is specific to the *S. mutans* strains in which the amount of glucose side chain of the serotype-specific polysaccharide antigen of *S. mutans* is reduced, and that the specific base sequence includes at least 12 contiguous bases of the base sequence of any of SEQ ID NO: 1 through 4.

Preferably, a kit for determining a serotype of *S. mutans* strains of the present invention includes an oligonucleotide that includes a base sequence, or a complementary sequence thereof, that includes at least 12 contiguous bases of the base sequence specific to serotype *c, e,* or *f S. mutans* strains.

Preferably, a kit for determining a serotype of *S. mutans* strains of the present invention further includes a reagent for obtaining chromosomal DNA or total RNA from the tissue sample of a subject.

Preferably, a kit for determining a serotype of *S. mutans* strains of the present invention is adapted so that the tissue sample is obtained from blood, saliva, or plaque.

Preferably, a kit for determining a serotype of *S. mutans* strains of the present invention further includes a reagent for separating bacteria from saliva or plaque.

Preferably, a kit for determining a serotype of *S. mutans* strains of the present invention further includes a reagent used for a PCR reaction.

Preferably, a kit for determining a serotype of *S. mutans* strains of the present invention further includes a reagent used for a hybridization reaction.

Preferably, a kit for determining a serotype of *S. mutans* strains of the present invention is adapted so that the oligonucleotide is labeled.

According to the foregoing construction, there is provided a kit for determining a serotype of *S. mutans* strains, whereby the kit excels in specifically detecting *S. mutans* strains in which the amount of glucose side chain of the serotype-specific polysaccharide antigen of *S. mutans* is reduced.

According to the foregoing construction, the presence of new serotype strains can be found by detecting genes that encode enzymes important for the biosynthesis of the serotype-specific polysaccharide antigen of the *S. mutans* new serotype strains.

More specifically, in order to solve the foregoing problems, a kit for determining a serotype of *S. mutans* strains according to the present invention includes an antibody that specifically binds to *S. mutans* strains in which the amount of glucose side chain of the serotype-specific polysaccharide antigen of *S. mutans* is reduced.

Preferably, a kit for determining a serotype of *S. mutans* strains according to the present invention includes an antibody that specifically binds to *S. mutans* strains of a new serotype.

According to the foregoing construction, there is provided a kit useful for the detection of *S. mutans* strains of a new serotype.

Preferably, a kit for determining a serotype of *S. mutans* strains according to the present invention includes an antibody that specifically binds to polysaccharide antigen of *S. mutans* strains in which the amount of glucose side chain of the serotype-specific polysaccharide antigen of *S. mutans* is reduced.

According to the foregoing construction, there is provided a kit for determining *S. mutans* strain that has a reduced amount of glucose side chain in the serotype-specific polysaccharide antigen of *S. mutans*, i.e., a serotype of *S. mutans* strains whereby the kit excels in specifically detecting *S. mutans* strains of a new serotype.

More specifically, in order to solve the foregoing problems, a method for producing an antibody according to the present invention is modified from conventional immunization methods.

In one aspect of the invention, a method for producing an antibody according to the present invention preferably includes the step of injecting the *S. mutans* strains, suspended in a phosphate-buffered saline, intravenously into the auricular vein of rabbits repeatedly for 5 consecutive days.

It is preferable that a method for producing an antibody according to the present invention further include the step of repeating immunization of the *S. mutans* strains, suspended in a phosphate-buffered saline, one week after the injecting step and for another 2 weeks, 5 times each week.

According to the foregoing method, there is provided a method for producing an antibody that specifically binds to *S. mutans* strains that have a reduced amount of glucose side chain in the serotype-specific polysaccharide antigen of *S. mutans,* i.e., *S. mutans* strains of a new serotype.

According to the foregoing construction, there is provided an antibody against a surface antigen of *S. mutans* of a new serotype, a feat unattainable with conventional immunization methods.

More specifically, a bacteria detecting tool of the present invention includes an oligonucleotide, fixed on a substrate, that include a base sequence, or a complementary sequence thereof, that is specific to the *S. mutans* strains of a new serotype.

It is preferable in a bacteria detecting tool of the present invention that the base sequence be a base sequence with at least 12 contiguous bases of a base sequence of a polynucleotide that encodes enzymes important for the biosynthesis of the polysaccharide antigen specific to *S. mutans* strains of a new serotype.

According to the foregoing construction, *S. mutans* strains of a new serotype included in a sample can be detected and identified.

According to the foregoing construction, *S. mutans* strains included in a sample can be tested comprehensively.

Additional objects, features, and strengths of the present invention will be made clear by the description below. Further, the advantages of the present invention will be evident from the following explanation in reference to the drawings.

### BRIEF DESCRIPTION OF THE DRAWINGS

Figs. 1A through 1E are diagrams showing reactions between antisera to TW295 and TW871 of *S. mutans* and RR extracts of TW295, TW871, and serotype *c, e,* and *f* strains.
Figs. 2A through 2C are diagrams showing results of Western blot detection of PA, CA-GTF, and CF-GTF expression in *S. mutans.*
Figs. 3A and 3B are diagrams showing phagocytosis rate of oral and blood isolates of *S. mutans.*
Fig. 4 is a diagram showing the presence of variants by comparing the base sequences of a first portion of *rgpF* gene in serotype *c* and serotype *k* strains of *S. mutans.*
Fig. 5 is a diagram, continuous from Fig. 4, showing the base sequence comparison of a first portion of rgpF gene in serotype *c* and serotype *k* strains of *S. mutans.*
Fig. 6 is a diagram, continuous from Fig. 5, showing the base sequence comparison of a first portion of *rgpF* gene in serotype *c* and serotype *k* strains of *S. mutans.*
Fig. 7 is a diagram, continuous from Fig. 6, showing the base sequence comparison of a first portion of *rgpF* gene in serotype *c* and serotype *k* strains of *S. mutans.*
Fig. 8 is a diagram, continuous from Fig. 7, showing the base sequence comparison of a first portion of *rgpF* gene in serotype *c* and serotype *k* strains of *S. mutans.*
Fig. 9 is a diagram, continuous from Fig. 8, showing the base sequence comparison of a first portion of *rgpF* gene in serotype *c* and serotype *k* strains of *S. mutans.*
Fig. 10 is a diagram, continuous from Fig. 9, showing the base sequence comparison of a first portion of *rgpF* gene in serotype *c* and serotype *k* strains of *S. mutans.*
Fig. 11 is a diagram, continuous from Fig. 10, showing the base sequence comparison of a first portion of *rgpF* gene in serotype *c* and serotype *k* strains of *S. mutans.*
Fig. 12 is a diagram, continuous from Fig. 11, showing the base sequence comparison of a first portion of *rgpF* gene in serotype *c* and serotype *k* strains of *S. mutans.*
Fig. 13 is a diagram, continuous from Fig. 12, showing the base sequence comparison of a first portion of *rgpF* gene in serotype *c* and serotype *k* strains of *S. mutans.*
Fig. 14 is a diagram aligning and comparing the base sequences of a first portion of *rgpF* gene in serotype *c* and serotype *k* strains of *S. mutans.*
Fig. 15 is a diagram showing optimal PCR conditions.
Fig. 16 is a diagram showing results of measurement on detection sensitivity of PCR amplification performed with serotype-specific primers, wherein genomic DNA extracted from MT8148 strain (*c*), TW295 strain (*k*), and FT1 strain (*k*) were diluted and used as templates.
Figs. 17A and 17B are diagrams showing results of PCR amplification performed with serotype-specific primers, wherein genomic DNA extracted from *S. mutans* strains of different serotypes and genomic DNA of different streptococci were used as templates.
Figs. 18A and 18B are diagrams showing results of PCR amplification performed with serotype-specific primers, wherein genomic DNA extracted from saliva samples was used as a template.
Figs. 19A and 19B are diagrams showing results of Western blot analysis on CA-GTF expression and CF-GTF expression in *S. mutans.*

### BEST MODE FOR CARRYING OUT THE INVENTION

The following will describe an embodiment of the present invention in detail. It should be appreciated however that the present invention is not limited in any ways by the following description.

### (1) Serotype k S. mutans strains

*S. mutans* is occasionally isolated from the blood of patients with IE. However, the mechanisms of invasion and survival remain to be elucidated. Two of four blood isolate strains from the blood of patients with bacteremia or infective endocarditis (strains TW295 and TW871) have been shown to be serologically untypable by an immunoprecipitation test.

Immunodiffusion analyses using antisera against these strains demonstrated that 2 of 100 isolates from the oral cavity of 100 subjects showed a positive reaction, while further analysis of 2500 oral isolates from 50 subjects revealed that all 50 isolates from a single subject were not reactive with anti-*c*, *e*, and *f*, though they were reactive with anti-TW295 and TW871 antisera. The oral isolates showed similar biological properties to the reference *S. mutans* strain MT8148, including high levels of sucrose dependent adhesion and cellular hydrophobicity, along with expressions of glucosyltransferases and a protein antigen c, PAc. The inventors designated these organisms as serotype *k*.

The inventors isolated and characterized a new *S. mutans* (serotype *k*) from human blood and oral cavity samples. The serological properties of serotype *k* strains were found to be similar to those of *gluA*-inactivated mutant strain of MT8148 (MT8148GD).

Serotype *k* oral isolates were less susceptible to phagocytosis, as were the *gluA*-inactivated mutant of strain MT8148 and blood isolates. These results indicate that *S. mutans* serotype *k* strains are present in the oral cavity of humans and may be able to survive longer in blood owing to their low susceptibility to phagocytosis.

MT8148GD showed significantly lower sucrose-dependent adhesion to a glass surface, sucrose-independent adhesion to saliva-coated hydroxyapatite, dextran-binding activity, and cell-associated glucosyltransferase (GTF) activity than its parent strain MT8148.

Western blot analysis revealed reduced GTFB and GTFC expression rates in serotype *k* strains as compared to MT8148, though the caries-inducing activity of MT8148GD and serotype *k* oral isolate in rats was similar to MT8148.

Thus, a glucose side chain defect in the serotype specific polysaccharide of *S. mutans* may be associated with its caries-inducing activity, though to a lesser extent than its other major surface proteins (for example, GTF, PAc).

In the present invention, antisera against serotype *k S. mutans* strains were obtained using techniques modified from conventional immunization techniques. The sequences of genes associated with the biosynthesis of serotype specific polysaccharide antigens of *S. mutans* strains, and a comparison was made between serotype *k* and known strains in the sequences. As a result, mutations were identified in the sequence of *rgpF* gene, a gene that is common to the serotype *k S. mutans* strains.

### (A) Polynucleotides

The present invention provides a polynucleotide that encodes a polypeptide that reduces the amount of glucose side chain of the polysaccharide antigen specific to *S. mutans*. As used herein, a "polypeptide that reduces the amount of glucose side chain of the polysaccharide antigen specific to *S. mutans"* refers to a "polypeptide that is associated with the biosynthesis of the polysaccharide antigen specific to serotype *k S. mutans* strains" or a "polypeptide that is associated with the biosynthesis of the serotype specific polysaccharide antigen of serotype *k S. mutans* strains". In one embodiment, the invention provides a polynucleotide, or a fragment thereof, having the base sequence of *any of* SEQ ID NO: 1 through 4. As used herein, the term "polynucleotide" refers to "nucleic acid" or "nucleic acid molecule," and it is intended to mean a polymer of nucleotides. As used herein, the term "base sequence" refers to a "nucleic acid sequence" or a "nucleotide sequence," and it is used to denote a sequence of deoxyribonucleotides (A, G, C, T). Further, as used herein, a "polynucleotide, or a fragment thereof, having the base sequence of *any of* SEQ ID NO: 1 through 4" refers to a polynucleotide, or a fragment thereof, that includes a sequence as represented by the deoxyribonucleotides A, G, C, and T of SEQ ID NO: 1 through 4.

A polynucleotide according to the present invention may be in the form of RNA (for example, mRNA) or DNA (for example, cDNA or genomic DNA). The DNA may be double stranded or single stranded. The single strand DNA or RNA may be a coding strand (also known as a sense strand) or a non-coding strand (also known as an anti-sense strand).

As used herein, the term "oligonucleotide" refers to a molecule of several to several ten nucleotides, and it is used interchangeably with "polynucleotide." The oligonucleotide is denoted by the number of nucleotides it contains. For example, the term dinucleotide (dimmer) or trinucleotide (trimer) is used to refer to oligonucleotides of short sequences, whereas long oligonucleotides are referred to as 30mers or 100mers. The oligonucleotide may be produced as a fragment of a polynucleotide, or alternatively chemically synthesized.

A fragment of a polynucleotide according to the present invention is a fragment of at least 12 nt (nucleotides), preferably about 15 nt, more preferably at least about 20 nt, further preferably at least about 30 nt, or even more preferably at least about 40 nt. By "a fragment at least 20 nt in length with the nucleotide sequence of *any of* SEQ ID NO: 1 through 4," for example, is intended fragments which include 20 or more contiguous bases from the nucleotide sequence of *any of* SEQ ID NO: 1 through 4. Since the nucleotide sequence of *any of* SEQ ID NO: 1 through 4 is provided by an embodiment of the present invention, generating such DNA fragments would be routine to the skilled artisan. For example, restriction endonuclease cleavage or shearing by sonication could easily be used to generate fragments of various sizes. Alternatively, such fragments could be generated synthetically. Suitable fragments (oligonucleotides) are synthesized with the Synthesizer Type 392 of Applied Biosystems Incorporated (ABI, 850 Lincoln Center Dr., Foster City, CA 94404).

The present invention relates to a polynucleotide of a sequence, or its complementary sequence, with at least 12 contiguous bases in the nucleotide sequence of *any of* SEQ ID NO: 1 through 4, regardless of whether it encodes a polypeptide associated with the biosynthesis of the polysaccharide antigen of specific to serotype *k S. mutans* strains. It will be apparent for a person ordinary skill in the art that the polynucleotide, even when it does not encode a polypeptide associated with the biosynthesis of the polysaccharide antigen specific to serotype *k S. mutans* strains, can be used as a primer for polymerase chain reaction (PCR), or a hybridization probe, for example. Since the polynucleotide of the present invention can be used for the PCR amplification specific to the polynucleotide that encodes a polypeptide associated with the biosynthesis of the polysaccharide antigen specific to serotype *k S. mutans* strains, it can be used as a hybridization probe that specifically hybridizes with the polynucleotide that encodes a polypeptide associated with the biosynthesis of the polysaccharide antigen specific to serotype *k S. mutans* strains. When the polynucleotide of the present invention does not encode a polypeptide associated with the biosynthesis of the polysaccharide antigen specific to serotype *k S. mutans* strains, the polynucleotide has the following applications. For example, the polynucleotide can be used for *in situ* hybridization (e.g., "FISH") to metaphase chromosomal spreads to provide precise chromosomal location, as described in Verma et al., Human Chromosomes: a manual of Basic Techniques, Pergamon Press, New York (1988). Another example is a tissue-specific Northern blot analysis for detecting mRNA expression concerning the polypeptide that is associated with the biosynthesis of the polysaccharide antigen specific to serotype *k S. mutans* strains.

By a portion of a polynucleotide of "at least 20 nt in length," for example, is intended 20 or more contiguous nucleotides from the nucleotide sequence of the reference polynucleotide. As indicated, such portions are useful diagnostically either as a probe according to conventional DNA hybridization techniques or as primers for amplification of a target sequence by the polymerase chain reaction (PCR).

In another aspect, the invention provides an isolated polynucleotide comprising a polynucleotide which hybridizes under stringent hybridization conditions to a polynucleotide of the present invention (for example, a polynucleotide with the base sequence of any of SEQ ID NO: 1 through 4), or to a portion of the polynucleotide of the invention. By "stringent hybridization conditions" is intended overnight incubation at 42°C in a solution comprising: 50% formamide, 5x SSC (150 mM NaCl, 15 mM trisodium citrate), 50 mM sodium phosphate (pH7.6), 5x Denhardt's solution, 10% dextran sulfate, and 20 µg/ml denatured, sheared salmon sperm DNA, followed by washing the filters in 0.1x SSC at about 65°C. By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably at least about 30-70 nt of the reference polynucleotide. These are useful as diagnostic probes and primers as discussed above and in more detail below.

As indicated, a polynucleotide of the present invention that encodes a polypeptide associated with the biosynthesis of the serotype-specific polysaccharide antigen of *S. mutans* strains of serotype *k* can include, but are not limited to, those encoding the amino acid sequence of the mature polypeptide, by itself, the coding sequence for the mature polypeptide and additional sequences, such as those encoding the amino acid leader sequence, such as a pre-, or pro- or prepro-protein sequence; the coding sequence of the mature polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing--including splicing and polyadenylation signals, an additional coding sequence which codes for additional amino acids, such as those which provide additional functionalities. Thus, the sequence encoding the polypeptide can be fused to a marker sequence, such as a sequence encoding a peptide which facilitates purification of the fused polypeptide. In certain preferred embodiments of this aspect of the invention, the marker amino acid sequence is a hexa-histidine peptide, such as the tag provided in a pQE vector (Qiagen, Inc.), among others, many of which are publicly and/or commercially available. As described in Gentz et al., Proc. Natl. Acad Sci. USA 86:821-824 (1989), for instance, hexa-histidine provides for convenient purification of the fusion protein. The "HA" tag is another peptide useful for purification which corresponds to an epitope derived from the influenza hemagglutinin (HA) protein, which has been described by Wilson et al., Cell 37:767 (1984). Other such fusion proteins include the polypeptide, or a fragment thereof, associated with the biosynthesis of the polysaccharide antigen specific to *S. mutans* of serotype *k*, and fused to Fc at the N- or C-terminus.

A polynucleotide of the present invention that encodes a polypeptide, or a fragment thereof, associated with the biosynthesis of the serotype-specific polysaccharide antigen of *S. mutans* strains of serotype *k* may be joined to a vector containing a selectable marker for propagation in a host. Generally, a plasmid vector is introduced in a precipitate, such as a calcium phosphate precipitate, or in a complex with a charged lipid. A DEAE-dextran method or electroporation method may be used, as will be described later. If the vector is a virus, it may be packaged *in vitro* using an appropriate packaging cell line and then transduced into host cells.

The present invention further relates to variants of a polynucleotide of the present invention, which encode a polypeptide associated with the biosynthesis of the serotype-specific polysaccharide antigen of *S. mutans* strains of serotype *k*. Variants can occur naturally, such as a natural allelic variant. By an "allelic variant" is intended one of several alternate forms of a gene occupying a given locus on a chromosome of an organism. Non-naturally occurring variants can be produced, e.g., using art-known mutagenesis techniques.

Such variants include those produced by nucleotide substitutions, deletions or additions. The substitutions, deletions or additions can involve one or more nucleotides. The variants can be altered in coding or non-coding regions or both. Alterations in the coding regions can produce conservative or non-conservative amino acid substitutions, deletions or additions.

As described above, a polynucleotide according to the present invention at least includes the base sequence of *any of* SEQ ID NO: 1 through 4, or comprises variants of these base sequences. Further, the base sequences of polynucleotides according to the present invention may be ligated to each other with a polynucleotide that encodes a suitable linker peptide.

In sum, an object of the present invention is to provide a polynucleotide that encodes a polypeptide associated with the biosynthesis of the polysaccharide antigen specific to serotype *k S. mutans* strains, and a polynucleotide (oligonucleotide) that is used to detect such polynucleotides. As such, the present invention is not just limited to the specific method of producing polynucleotides, or other disclosure of the present invention. It should be appreciated that the polynucleotide that encodes a polypeptide associated with the biosynthesis of the polysaccharide antigen of serotype *k S. mutans* strains, and the polynucleotide (oligonucleotide) that is used to detect such a polynucleotide fall within the scope of the present invention even when they are obtained by methods other than those described in the present invention.

### (B) Polypeptide

The inventors of the present invention found *S. mutans* strains of a novel serotype (type *k*), and found that there were variants in *rgpF,* which is one of the enzymes associated with the biosynthesis of the polysaccharide antigen that defines this serotype.

The term "polypeptide" is considered synonymous to "peptide" and "protein" and each term can be used interchangeably. Further, a "fragment" of the polypeptide refers to a partial fragment of the polypeptide. A polypeptide according to the present invention may be isolated from a natural source or chemically synthesized.

By "isolated" polypeptide or protein is intended a polypeptide or protein removed from its native environment. For example, recombinantly produced polypeptides and proteins expressed in host cells are considered isolated for purposes of the invention as are native or recombinant polypeptides and proteins which have been substantially purified by any suitable technique.

Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

In one aspect of the invention, the present invention provides RgpF polypeptide of serotype *k S. mutans* strains. As used herein, "RgpF polypeptide of the serotype *k S. mutans* strains" refers to RgpF polypeptide as an enzyme that is associated with the biosynthesis of the polysaccharide antigen of the novel serotype *k S. mutans* strains.

In one embodiment of the invention, the present invention provides a polypeptide that is encoded by the base sequence of *any of* SEQ ID NO: 1 through 4. Preferred polypeptides according to the present invention include a polypeptide comprising: a mature polypeptide, an extracellular domain, a transmembrane domain, an intracellular domain, or an extracellular and intracellular domain with all or part of the transmembrane domain deleted.

Thus, the invention further provides variations of the RgpF polypeptide of serotype *k S. mutans* strains, which show substantial RgpF polypeptide activity of serotype *k S. mutans* strains. In one embodiment of the invention, the present invention provides a polypeptide mutant that shows RgpF polypeptide activity of serotype *k S. mutans* strains, wherein the mutant has substitution, addition, or deletion of one or more amino acids in the polypeptide encoded by the base sequence of *any of* SEQ ID NO: 1 through 4.

As used herein, the "RgpF polypeptide activity of serotype *k S. mutans* strains" refers to characteristics of RgpF polypeptide that is associated with the biosynthesis of the polysaccharide antigen that does not exist in *S. mutans* strains of known serotypes, i.e., the polysaccharide antigen specific to serotype *k S. mutans.*

Such mutants include deletions, insertions, inversions, repeats, and type substitutions (for example, substituting one hydrophilic residue for another, but not strongly hydrophilic for strongly hydrophobic as a rule). Small changes or such "neutral" amino acid substitutions will generally have little effect on activity.

It will be recognized in the art that some amino acid sequences of the polypeptide can be varied without significant effect on the structure or function of the polypeptide. It is also known that such a mutant with no significant structural or functional change occurs not only in artificially modified proteins but in nature as well.

It is easy for a person ordinary skill in the art to modify one to several amino acids in the amino acid sequence of a protein using a conventional technique. For example, by a conventional point mutation introducing method, any base of a polynucleotide that encodes a protein can be mutated. Further, with a primer that is designed to correspond to an arbitrary site of a polynucleotide that encodes a protein, a deletion mutant or an addition mutant can be produced. Further, with the method described in the present invention, whether or not the mutant has desired activities can easily be evaluated.

The mutants preferably include those produced by substitutions, deletions, or additions of amino acid, which may be conservative or non-conservative. Especially preferred among these are silent substitutions, additions and deletions. Also especially preferred are conservative substitutions. These do not alter the RgpF polypeptide activity of properties and activities of serotype *k S. mutans* strains.

Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. As indicated in detail above, further guidance concerning which amino acid changes are likely to be phenotypically silent (i.e., are not likely to have a significant deleterious effect on a function) can be found in Bowie, J. U., et al., "Deciphering the Message in Protein Sequences: Tolerance to Amino Acid Substitutions," Science 247:1306-1310 (1990) (herein incorporated by reference).

In one aspect of the invention, the polypeptide may be expressed in a modified form, such as a fusion protein. For instance, a region of additional amino acids, particularly charged amino acids, may be added to the N-terminus of the polypeptide to improve stability and persistence in the host cell, during purification or during subsequent handling and storage.

In one aspect of the invention, the RgpF polypeptide of the serotype *k S. mutans* strains according to the present invention may have a tag label (tag sequence or marker sequence), which is a coding sequence of a peptide that facilitates purification of the fused polypeptide for example, appended at the N terminus or C terminus. Such sequences can be removed before the final preparation stage of the polypeptide. In certain preferred embodiments of this aspect of the invention, the marker amino acid sequence is a hexa-histidine peptide, such as the tag provided in a pQE vector (Qiagen, Inc.), among others, many of which are publicly and/or commercially available. As described in Gentz et al., Proc. Natl. Acad Sci. USA 86:821-824 (1989), for instance, hexa-histidine provides for convenient purification of the fusion protein. The "HA" tag is another peptide useful for purification which corresponds to an epitope derived from the influenza hemagglutinin (HA) protein, which has been described by Wilson et al., Cell 37:767 (1984). Other such fusion proteins include rgpF polypeptide, or a fragment thereof, of serotype *k S. mutans* strains according to embodiments of the present invention, in which Fc is fused with the RgpF polypeptide at the N terminus or C terminus.

The RgpF polypeptide of the serotype *k S. mutans* strains according to the present invention may be produced by recombination or chemically synthesized.

Recombination can be performed by methods known in the art, using vectors and cells describe below, for example.

The synthetic peptide can be synthesized by a known method of chemical synthesis. For instance, Houghten has described a simple method for synthesis of large numbers of peptides, such as 10-20 mg of 248 different 13 residue peptides representing single amino acid variants of a segment of the HA1 polypeptide which were prepared and characterized in less than four weeks. Houghten, R. A., Proc. Natl. Acad. Sci. USA 82:5131-5135 (1985). This "Simultaneous Multiple Peptide Synthesis (SMPS)" process is further described in U.S. Pat. No. 4,631,211 to Houghten et al., (1968). In this procedure the individual resins for the solid-phase synthesis of various peptides are contained in separate solvent-permeable packets, enabling the optimal use of the many identical repetitive steps involved in solid-phase methods. A completely manual procedure allows 500-1000 or more syntheses to be conducted simultaneously. Houghten et al., supra, at 5134.

In one aspect of the invention, the present invention relates to a polypeptide with an amino acid sequence of the epitope-bearing portion of the RgpF polypeptide of serotype *k S. mutans* strains of the present invention. The polypeptide with an amino acid sequence of the epitope-bearing portion of the RgpF polypeptide of serotype *k S. mutans* strains of the present invention may include portions of such polypeptides with at least six, seven, eight, nine, or ten amino acids, although epitope-bearing polypeptides of any length up to and including the entire amino acid sequence of the RgpF polypeptide of the serotype *k S. mutans* strains of the invention encoded by the base sequence of *any of* SEQ ID NO: 1 through 4 also are included in the invention.

The epitope portion of the RgpF polypeptide of the serotype *k S. mutans* strains of the present invention is identified by a method known in the art. For instance, Geysen et al., (1984), supra, discloses a procedure for rapid concurrent synthesis on solid supports of hundreds of peptides of sufficient purity to react in an enzyme-liked immunosorbent assay. Interaction of synthesized peptides with antibodies is then easily detected without removing them from the support. In this manner a peptide bearing an immunogenic epitope of a desired protein may be identified routinely by one of ordinary skill in the art. For instance, the immunologically important epitope in the coat protein of foot-and-mouth disease virus was located by Geysen et al. with a resolution of seven amino acids by synthesis of an overlapping set of all 208 possible hexapeptides covering the entire 213 amino acid sequence of the protein. Then, a complete replacement set of peptides in which all 20 amino acids were substituted in turn at every position within the epitope were synthesized, and the particular amino acids conferring specificity for the reaction with antibody were determined. Thus, peptide analogs of the epitope-bearing peptides of the invention can be made routinely by this method. U.S. Pat. No. 4,708,781 to Geysen (1987) further describes this method of identifying a peptide bearing an immunogenic epitope of a desired protein.

An "immunogenic epitope" is defined as a part of a protein that elicits an antibody response when the whole protein is the immunogen. These immunogenic epitopes are believed to be confined to a few loci on the molecule. On the other hand, a region of a protein molecule to which an antibody can bind is defined as an "antigenic epitope." The number of immunogenic epitopes of a protein generally is less than the number of antigenic epitopes. See, for instance, Geysen, H. M. et al., Proc. Natl. Acad. Sci. USA 81:3998-4002 (1984).

Antigenic epitope-bearing peptides of the invention are therefore useful to raise antibodies, including monoclonal antibodies, that bind specifically to a polypeptide of the invention. Thus, a high proportion of hybridomas obtained by fusion of spleen cells from donors immunized with an antigen epitope-bearing peptide generally secrete antibody reactive with the native protein. The antibodies raised by antigenic epitope-bearing peptides or polypeptides are useful to detect the mimicked protein, and antibodies to different peptides may be used for tracking the fate of various regions of a protein precursor which undergoes posttranslation processing. The peptides and anti-peptide antibodies may be used in a variety of qualitative or quantitative assays for the mimicked protein, for instance in competition assays since it has been shown that even short peptides (e.g., about 9 amino acids) can bind and displace the larger peptides in immunoprecipitation assays. See, for instance, Wilson, I. A. et al., Cell 37:767-778 (1984) at 777. The anti-peptide antibodies of the invention also are useful for purification of the mimicked protein, for instance, by adsorption chromatography using methods well known in the art.

Antigenic epitope-bearing peptides of the invention designed according to the above guidelines preferably contain a sequence of at least seven, more preferably at least nine and most preferably between about 15 to about 30 amino acids contained within the amino acid sequence of a polypeptide of the invention. However, peptides or polypeptides comprising a larger portion of an amino acid sequence of a polypeptide of the invention, containing about 30 to about 50 amino acids, or any length up to and including the entire amino acid sequence of a polypeptide of the invention, also are considered epitope-bearing peptides of the invention and also are useful for inducing antibodies that react with the mimicked protein. Preferably, the amino acid sequence of the epitope-bearing peptide is selected to provide substantial solubility in aqueous solvents (i.e., the sequence includes relatively hydrophilic residues and highly hydrophobic sequences are preferably avoided); and sequences containing proline residues are particularly preferred.

The epitope-bearing peptides of the invention may be produced by any conventional means for making peptides including recombinant means using polynucleotides of the invention. For instance, a short epitope-bearing amino acid sequence may be fused to a larger polypeptide which acts as a carrier during recombinant production and purification, as well as during immunization to produce anti-peptide antibodies. Epitope-bearing peptides also may be synthesized using known methods of chemical synthesis.

Epitope-bearing peptides of the invention are used to induce antibodies according to methods well known in the art. See, for instance, Chow, M. et al., Proc. Natl. Acad. Sci. USA 82: 910-914; and Bittle, F. J. et al., J. Gen. Virol. 66: 2347-2354 (1985). Generally, animals may be immunized with free peptide; however, anti-peptide antibody titer may be boosted by coupling of the peptide to a macromolecular carrier, such as keyhole limpet hemacyanin (KLH) or tetanus toxoid. For instance, peptides containing cysteine may be coupled to carrier using a linker such as m-maleimidobenzoyl-N-hydroxysuccinimide ester (MBS), while other peptides may be coupled to carrier using a more general linking agent such as glutaraldehyde. Animals such as rabbits, rats and mice are immunized with either free or carrier-coupled peptides, for instance, by intraperitoneal and/or intradermal injection of emulsions containing about 100 µg peptide or carrier protein and Freund's adjuvant. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

### (C) Vectors and Cells

The present invention also relates vectors which are used to produce RgpF polypeptides of serotype *k S. mutans* strains of the present invention. Vectors according to the present invention may be vectors used for *in vitro* translation, or vectors used for recombination and expression.

The present invention also relates to host cells that are transformed with a recombinant construct that includes a polynucleotide according to the present invention, the host cells being used in recombination to generate RgpF polypeptides of serotype *k S. mutans* strains of the present invention. The invention also relates to a process for producing RgpF polypeptides, or fragments thereof, of serotype *k S. mutans* strains by recombinant techniques.

Recombinant constructs may be introduced into host cells using well known techniques such as infection, transduction, transfection, electroporation and transformation. The vector may be, for example, a phage, plasmid, viral or retroviral vector. Retroviral vectors may be replication competent or replication defective. In the latter case, viral propagation generally will occur only in complementing host cells.

The polynucleotides that encode the target polypeptides to be expressed can be obtained by a method known in the art. In order to obtain a partial sequence of a known protein, primers are designed for the target portion and amplified by PCR. Resulting corresponding polynucleotides are then introduced into the expression vector. In this way, the sequence of interest can easily be obtained in the host cells.

Preferred are vectors comprising cis-acting control regions to the polynucleotide of interest. Appropriate trans-acting factors may be supplied by the host, supplied by a complementing vector or supplied by the vector itself upon introduction into the host.

In certain preferred embodiments in this regard, the vectors provide for specific expression, which may be inducible and/or cell type-specific. Particularly preferred among such vectors are those inducible by environmental factors that are easy to manipulate, such as temperature and nutrient additives.

Expression vectors useful in the present invention include chromosomal-, episomal- and virus-derived vectors, e.g., vectors derived from bacterial plasmids, bacteriophage, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof, such as cosmids and phagemids.

The DNA insert should be operatively linked to an appropriate promoter, such as the phage lambda PL promoter, the *E. coli* lac, trp and tac promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will include a translation initiating codon at the beginning and a termination codon appropriately positioned at the end of the polypeptide to be translated.

The expression vectors will preferably include at least one selectable marker. Such markers include dihydrofolate reductase or neomycin resistance for eukaryotic cell culture and tetracycline or ampicillin resistance genes for culturing in *E. coli* and other bacteria. Representative examples of appropriate hosts include bacterial cells, such as *E. coli, Streptomyces* and *Salmonella tphimurium* cells; fungal cells, such as yeast cells; insect cells such as Drosophila S2and Spodoptera Sf9 cells; animal cells such as CHO, COS and Bowes melanoma cells; and plant cells. Appropriate culture media and conditions for the above-described host cells are known in the art.

In a preferred embodiment, vectors according to the present invention can express the rgpF polypeptides of serotype *k S. mutans* strains of the present invention in bacteria (*E. coli* in particular). Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia.

Among known bacterial promoters suitable for use in the present invention include the E. coli lacI and lacZ promoters, the T3 and T7 promoters, the gpt promoter, the lambda PR and PL promoters and the trp promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-1 promoter.

In another embodiment, vectors according to the present invention can express the rgpF polypeptides of serotype *k S. mutans* strains of the present invention in higher eukaryotic cells. Transcription of the DNA by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

Among preferred eukaryotic vectors are pWLNEO, pSV2CAT, pOG44, pXT1 and pSG available from Stratagene; pSVK3, pBPV, pMSG and pSVL available from Pharmacia, and pA2 available from Qiagen. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods in Molecular Biology, which is hereby incorporated by reference.

Transcription of the DNA by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide or they may be heterologous signals.

Thus, the polypeptide may be expressed in a modified form, such as a fusion protein, and may include not only secretion signals but also additional heterologous functional regions. For instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP A O 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as, hIL-5- has been fused with Fc portions for the purpose of high-throughput screening assays to identify antagonists of hIL-5. See, D. Bennett et al., Journal of Molecular Recognition, Vol. 8 52-58 (1995) and K. Johanson et al., The Journal of Biological Chemistry, Vol. 270, No. 16, pp 9459-9471 (1995).

The RgpF peptides of the serotype *k S. mutans* strains can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

As described above, vectors according to the present invention include polynucleotides according to the present invention. Therefore, it should be appreciated that vectors other than the expression vector also fall within the technical scope of the present invention.

Further, cells according to the present invention at least include vectors of the present invention. That is, primary cultured cells other than commercially available cells also fall within the technical scope of the present invention.

More specifically, an object of the present invention is to provide vectors that include a polynucleotide of the present invention, and cells including such vectors. As such, the present invention is not limited to the specific types of vectors and cells, the specific methods of vector preparation, and the specific methods of introduction described above. Therefore, it should be appreciated that the scope of the present invention also includes vectors and cells other than those described above, as well as cells and vectors that are obtained by methods other than the foregoing methods.

### (D) Bacteria

The present invention provides isolates of serotype *k S. mutans* strains. Preferably, serotype *k S. mutans* strains of the present invention are isolated from biological samples, using antibodies that specifically bind to serotype *k S. mutans* strains, as described below. Preferable examples of the serotype *k S. mutans* strains include, but are not limited to, 2 blood isolates (TW295 and TW871), and 154 oral isolates (FT1 through FT51, SU1 through SU51, YK1 through YK50, and AT1 through YT1). By "biological sample" is intended any biological sample obtained from an individual, body fluid, cell line, tissue culture, or other source which contains serotype *k S. mutans* strain proteins, or DNA or mRNA thereof. As indicated, biological samples include body fluids (such as blood, saliva, dental plaque, sera, plasma, urine, synovial fluid and spinal fluid) which contain serotype *k S. mutans* strain protein, or tissue sources found to express serotype *k S. mutans* strain protein. By "tissue sample" is intended biological samples obtained from tissue sources. Methods for obtaining tissue biopsies and body fluids from mammals are well known in the art. Where the biological sample is to include mRNA, a tissue biopsy is the preferred source. As used herein, the term "sample" refers to not only biological samples and tissue samples as defined above, but genomic DNA samples and/or total RNA samples extracted from the biological samples and tissue samples.

In one aspect of the invention, the present invention provides *S. mutans* strains that have a reduced amount of glucose side chain in the serotype-specific polysaccharide antigen of *S. mutans.* As used herein, "a reduced amount of glucose side chain of the polyscaaharide antigen specific to serotype *c, e,* or *f* of *S. mutans"* is intended to mean that the glucose side chain is simply undetectable with known antibodies specific to serotype *c, e,* or *f* strain of *S. mutans.* As such, it does not mean lack of the glucose side chain of the polysaccharide antigen specific to serotype *c, e,* or *f* strains of *S. mutans.* Rather, it will be apparent to the skilled artisan that it is intended to mean a significantly reduced level of antigenicity against known antibodies specific to serotype *c*, *e*, or *f* strain of *S. mutans.*

### (E) Antibodies

The present invention provides antibodies that specifically bind to strains of a serotype with a reduced amount of glucose side chain in the polysaccharide antigen specific to serotype *c, e,* or *f* strain of *S. mutans.* Preferably, the invention provides antibodies that specifically bind to serotype *k S. mutans* strains. As used herein, the term "antibodies" refers to immunoglobulins (IgA, IgD, IgE, IgG, IgM, and Fab fragments, F(ab')₂ fragments, and Fc fragments thereof), non-limiting examples of which include polyclonal antibodies, monoclonal antibodies, single-chain antibodies, anti-ideotype antibodies, and humanized antibodies. Antibodies of the present invention, which have been unattainable with conventional methods were obtained by making unique modifications. Antibodies of the present invention are produced with mutant strains of known *S*. *mutans* strains of serotypes other than serotype *k,* using a known glucose side chain deletion method. Further, antibodies of the present invention are produced by using polypeptides of the present invention as antigens. Antibodies of the present invention may be useful in the detection and/or diagnosis of serotype *k S. mutans* strains.

As used herein, "antibodies against serotype *k S. mutans* strains" includes intact molecules, and antibody fragments (for example, Fab and F(ab')₂), that can specifically bind to the antigens of serotype *k S. mutans* strains. Fab and F(ab')₂ fragments lack the Fc fragment of intact antibody, clear more rapidly from the circulation, and may have less non-specific tissue binding than an intact antibody (Wahl et al., J. Nucl. Med. 24:316-325 (1983)). Thus, such fragments are also preferable.

Alternatively, additional antibodies capable of binding to the antigens of the serotype *k S. mutans* strains may be produced in a two-step procedure through the use of anti-idiotypic antibodies. Such a method makes use of the fact that antibodies are themselves antigens, and that, therefore, it is possible to obtain an antibody which binds to a second antibody. In accordance with this method, serotype *k S. mutans* strain-specific antibodies are used to immunize an animal, preferably a mouse. The splenocytes of such an animal are then used to produce hybridoma cells, and the hybridoma cells are screened to identify clones which produce an antibody whose ability to bind to the serotype *k S. mutans*-specific antibody can be blocked by the serotype *k S. mutans* strain-specific antigen. Such antibodies comprise anti-idiotypic antibodies to the serotype *k S. mutans* strain-specific antibody and can be used to immunize an animal to induce formation of further serotype *k S. mutans* strain-specific antibodies.

It will be appreciated that Fab and F(ab')₂ and other fragments of the antibodies of the present invention may be used according to the methods disclosed herein. Such fragments are typically produced by proteolytic cleavage, using enzymes such as papain (to produce Fab fragments) or pepsin (to produce F(ab')₂ fragments). Alternatively, serotype *k S*. *mutans* strain-binding fragments can be produced through the application of recombinant DNA technology or through synthetic chemistry.

Where *in vivo* imaging is used to detect enhanced levels of serotype *k S. mutans* strains for diagnosis in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. See, for review, Morrison, Science 229:1202 (1985); Oi et al., BioTechniques 4:214 (1986); Cabilly et al., U.S. Pat. No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).

As described above, antibodies according to the present invention at least include antibody fragments (for example, Fab and F(ab')₂), that recognize serotype *k S. mutans* strains of the present invention. Therefore, it should be appreciated that the present invention also includes immunoglobulins that comprise (i) antibody fragments that recognize serotype *k S. mutans* strains of the present invention, and (ii) Fc fragments of different antibody molecules.

More specifically, since an object of the present invention is to provide antibodies that recognize serotype *k S*. *mutans* strains of the present invention, the invention is not limited to the specific types of immunoglobulins (IgA, IgD, IgE, IgG, or IgM), or the specific methods of producing chimeric antibodies and peptide antibodies, etc. Therefore, it should be appreciated that antibodies that are obtained by methods other than those described above also fall within the scope of the present invention.

### (2) Methods of Detection and Diagnosis of Serotype k S. mutans Strains

A detection method and diagnosis method of a serotype of *S. mutans* according to the present invention are methods for detecting and diagnosing serotype *k S. mutans* strains that may be present in a subject.

Detection of infective factors or other molecules (such as metabolites, nucleic acids, and proteins) that are used as indices of disease is a basic element in the diagnosis and treatment of medical disorders as well as research. Currently, many methodologies are available for detection. These methodologies can be divided into two types of diagnostic assays: diagnostic assays for genetic materials, such as nucleic acids, that encode constituents of disease-causing factors; and diagnostic assays based on antibodies against proteins whose constituents are either disease-causing factors or byproducts of disease.

The present invention provides a method for diagnosing disorders associated with serotype *k S. mutans* strains, whereby a polynucleotide that encodes an enzyme associated with the biosynthesis of the polysaccharide antigen specific to serotype *k S. mutans* strains is detected in a biological sample, or more specifically, in the tissues, cells, or body fluids obtained from a subject.

### (A) Detection Method and Diagnosis Method for Nucleic Acids

The assay for nucleic acids may be as simple as Northern blot hybridization that uses a radio-labeled probe for the detection of polynucleotides (nucleic acid molecules), or as sophisticated as polymerase chain reaction (PCR), which amplifies a minute amount of nucleic acid sequence to the extent where is can be detected by hybridization techniques. The nucleotide probe can be labeled with commercially available dyes, or alternatively, an enzyme label, fluorescent label, a chemiluminescent label, or a radioactive label may be used therefor. These probes can be used for hybridization to detect expression of genes, or related sequences (genes of common constituents), in cells or tissue samples, or for the screening of sera or tissue samples obtained from possible carrier humans of the disease caused by the infection. The primers can be prepared by PCR reaction using reverse transcriptase or DNA polymerase, and can be used to detect polynucleotides that are present in a minute amount in the tissue or solution.

The polymerase chain reaction (PCR) has become a tool of major importance finding applications in cloning, analysis of genetic expression, DNA sequencing, genetic mapping, drug discovery, and the like, e.g., Innis et al. in PCR Protocols A guide to Methods and Applications, Academic Press, San Diego (1990); and U.S. Pat. Nos. 4, 683,195, 4,683.

The present invention provides a method for detecting serotype *k S. mutans* strains, the method including the step of carrying out a PCR reaction with a primer, for which an oligonucleotide of a base sequence, or a complementary sequence thereof, of at least 12 bases of the base sequence of any of SEQ ID NO: 1 through 4 is used. PCR reagents and methodology are available from Perkin Elmer, 761 Main Ave., Norwalk, Conn. 06850; or Roche Molecular Systems, 1145 Atlantic Ave., Suite 100, Alameda, CA 94501. Preferably, the PCR reaction is performed with 20 ml of PCR reaction solution (comprising 5 µl of 20 ng/µl genomic DNA, 2 µl of dNTP mixture, 2 µl of 10 ×PCR buffer, 1 µl of forward primer (20 pmol/µl), 1 µl of reverse primer (20 pmol/µl), 0.1 µl of AmpliTaq Gold, and 8.9 µl of MilliQ water). The PCR reaction is preferably performed with the following cycling parameters: one cycle consisting of 94°C for 5 minutes; 30 cycles consisting of 94°C for 30 seconds, 60°C for 30 seconds, and 72°C for 30 seconds, and then one cycle consisting of 72°C for 7 minutes. The conditions of the PCR reaction are not limited to these examples. As used herein, "at least 12" means an integer of 12, 13, 14, 15, or greater. According to the present invention, whether *S. mutans* strains of serotype *k* are present in a sample obtained from a subject can be determined both effectively and efficiently.

Traditionally, PCR and probe hybridization processes have been performed as separate operations. However, it is highly desirable to perform both the PCR and the probe hybridization reactions in a combined manner. There are several advantages realized by combining the PCR and the probing process: (i) only a single reagent addition step is required, thereby allowing the combined reactions to proceed without having to open up the reaction tube, thereby reducing the opportunity for sample mix-up and sample contamination; (ii) fewer reagents are needed; (iii) fewer reagent addition steps are required, making automation more straight forward; and, (iv) in the case of in situ methods, there is no requirement to take apart a sample containment assembly used to protect the integrity of the cellular sample during thermocycling. One existing method which combines PCR with hybridization probing in a single reaction is the technique know as "Taqman", e.g., Holland et al, Proc. Natl. Acad. Sci. USA 88: 7276-7280 (1991).

"*In situ* PCR" refers to PCR amplification performed in fixed cells, such that specific amplified nucleic acid is substantially contained within a cell or subcellular structure which originally contained the target nucleic acid sequence. The cells may be in aqueous suspension or may be part of a tissue sample, e.g., histochemical section, or a cytochemical smear. As used herein, the term "histochemical section" refers to a solid sample of biological tissue which has been frozen or chemically fixed and hardened by embedding in a wax or plastic, sliced into a thin sheet (typically several microns thick), and attached to a solid support, e.g., a microscope slide, and the term "cytochemical smear" refers to a suspension of cells, e.g., blood cells, which has been chemically fixed and attached to a microscope slide. Preferably, the cells will have been rendered permeable to PCR reagents by proteinase digestion, by liquid extraction with a surfactant or organic solvent, or other like permeablization methods. Preferably, the cells are made permeable to PCR reagents by a method such as proteinase digestion, liquid extraction using a surfactant or an organic solvent, or permeabilization.

As used herein, the term "fixed cells" refers to a sample of biological cells which has been chemically treated to strengthen cellular structures, particularly membranes, against disruption by solvent changes, temperature changes, mechanical stress or drying. Cells may be fixed either in suspension our as part of a tissue sample. Cell fixatives generally are chemicals which crosslink the protein constituents of cellular structures, most commonly by reacting with protein amino groups. Preferred fixatives include buffered formalin, 95% ethanol, fomaldehyde, paraformaldehyde, and glutaraldehyde. The permeability of fixed cells can be increased by treatment with proteinases, or with surfactants or organic solvents which dissolve membrane lipids.

The present invention provides a method for detecting serotype *k S. mutans* strains, the method including the step of carrying out a hybridization reaction with a probe, for which an oligonucleotide of a base sequence, or a complementary sequence thereof, of at least 12 bases of the base sequence of any of SEQ ID NO: 1 through 4 is used. According to the present invention, whether *S. mutans* strains of serotype *k* are present in a sample obtained from a subject can be determined both effectively and efficiently.

An oligonucleotide of the present invention is used for the genetic mapping by in situ hybridization with chromosomes. The oligonucleotide is therefore useful, for example, as a probe used in Northern blot analysis to detect expression of serotype *k S. mutans* strains in human tissues. As detailed below, detecting changes in the gene expression of serotype *k S. mutans* strains in a specific tissue can reveal a specific disease and/or disorder.

In a preferred embodiment, an oligonucleotide of the present invention is labeled. Suitable labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labeled antibody/substrate reaction. Besides enzymes, other suitable labels include radioisotopes, such as iodine (¹²⁵I, ¹²¹I), carbon (¹⁴C), sulphur (³⁵S), tritium (³H), indium (¹¹²In), and technetium (^{99m}Tc), and fluorescent labels, such as fluorescein and rhodamine, and biotin.

### (B) Detection Method and Diagnosis Method for Proteins

The assay for proteins may be a method that includes either a binding reaction of the molecules (common antibodies) with tested proteins, or a reaction of an enzyme with its target molecule, whereby the enzyme is activated and the substrate that shows a detectable color change is cleaved. Many binding assays include dye-, enzyme-, radioactive-, or fluorescent-labels to enhance detection. The antibodies against the proteins can be obtained from patients, immunized animals, or antigen-specific monoclonal cell lines. These antibody assays include sandwich ELASA assay, Western blotting, radioimmuno assay, and immunodiffusion assay. Other assays use molecules such as avidin and biotin to immobilize and detect molecules. Techniques for preparing such reagents, and methods used for such techniques are known to the skilled artisan.

The present invention provides a method for detecting *S. mutans* strains of serotype *k,* the method including the step of performing an immunization reaction using an antibody that specifically binds to strains of a serotype in which the amount of glucose side chain of the polysaccharide specific to serotype *c, e,* or *f* strains of *S. mutans* is reduced. As used herein, "immunization reaction" means, but is not limited to, sandwich ELASA assay, Western blotting, radioimmuno assay, and immunodiffusion assay. According to the present invention, whether *S. mutans* strains of serotype *k* are present in a sample obtained from a subject can be determined both effectively and efficiently.

As described above, according to the present invention, the presence or absence of *S. mutans* strains that are less susceptible to the phagocytosis of the polymorphonuclear leukocytes can be determined upon entry into blood. Further, by combining with identification methods for other major causative bacteria, the number of potential patients requiring IE prevention can be reduced. In addition, since the amount of antibiotics used can be reduced, emergence of resistant bacteria due to use of antibiotics can be suppressed.

Further, the present invention provides a method for diagnosing *S. mutans* strains of serotype *k*, wherein the method uses the antibody that specifically binds to the serotype *k S. mutans* strains described herein. The antibody assays include sandwich ELASA assay, Western blotting, radioimmuno assay, and immunodiffusion assay. Other assays use molecules such as avidin and biotin to immobilize and detect molecules. Techniques for preparing such reagents, and methods used for such techniques are known to the skilled artisan.

Assaying serotype *k S. mutans* strain protein levels in a biological sample can occur using any art-known method. Preferred for assaying serotype *k S. mutans* strain protein levels in a biological sample are antibody-based techniques. For example, serotype *k S. mutans* strain protein expression in tissues can be studied with classical immunohistological methods. In these, the specific recognition is provided by the primary antibody (polyclonal or monoclonal) but the secondary detection system can utilize fluorescent, enzyme, or other conjugated secondary antibodies. As a result, an immunohistological staining of tissue section for pathological examination is obtained.

Other antibody-based methods useful for detecting serotype *k S. mutans* strain protein levels include immunoassays, such as the enzyme linked immunosorbent assay (ELISA) and the radioimmunoassay (RIA). For example, serotype *k S. mutans* strain-specific monoclonal antibodies can be used both as an immunoabsorbent and as an enzyme-labeled probe to detect and quantify the serotype *k S. mutans* strain protein. The amount of serotype *k S. mutans* strain protein present in the sample can be calculated by reference to the amount present in a standard preparation using a linear regression computer algorithm. Such an ELISA for detecting a tumor antigen is described in Iacobelli et al., Breast Cancer Research and Treatment 11:19-30 (1988). In another ELISA assay, two distinct specific monoclonal antibodies can be used to detect serotype *k S. mutans* strain protein in a body fluid. In this assay, one of the antibodies is used as the immunoabsorbent and the other as the enzyme-labeled probe.

Suitable enzyme labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labeled antibody/substrate reaction. Besides enzymes, other suitable labels include radioisotopes, such as iodine (¹²⁵I, ¹²¹I), carbon (¹⁴C), sulphur (³⁵S), tritium (³H), indium (¹¹²In), and technetium (^{99m}Tc), and fluorescent labels, such as fluorescein and rhodamine, and biotin.

In addition to assaying serotype *k S. mutans* strain protein levels in a biological sample obtained from an individual, serotype *k S. mutans* strain protein can also be detected *in vivo* by imaging. Antibody labels or markers for *in vivo* imaging of serotype *k S. mutans* strain protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A serotype *k S. mutans* strain protein-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously or intraperitoneally) into the mammal to be examined for disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ^{99m}Tc. The labeled antibody or antibody fragment will then preferentially accumulate at the location of cells which contain serotype *k S. mutans* strain protein. *In vivo* tumor imaging is described in S. W. Burchiel et al., "Immunopharmacokinetics of Radiolabelled Antibodies and Their Fragments" (Chapter 13 in Tumor Imaging: The Radiochemical Detection of Cancer, S. W. Burchiel and B. A. Rhodes, eds., Masson Publishing Inc. (1982)).

Further suitable labels for the serotype *k S. mutans* strain protein-specific antibodies of the present invention are provided below. Examples of suitable enzyme labels include malate dehydrogenase, staphylococcal nuclease, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase.

Examples of suitable radioisotopic labels include ³H, ¹¹¹In, ¹²⁵I, ¹³¹I, ³²P, ³⁵S, ¹⁴C, ⁵¹Cr, ⁵⁷To, ⁵⁸Co, ⁵⁹Fe, ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. ¹¹¹In is a preferred isotope where *in vivo* imaging is used since its avoids the problem of dehalogenation of the ¹²⁵I or ¹³¹I-labeled monoclonal antibody by the liver. In addition, this radionucleotide has a more favorable gamma emission energy for imaging (Perkins et al., Eur. J. Nucl. Med. 10:296-301 (1985); Carasquillo et al., J. Nucl. Med. 28:281-287 (1987)). For example, ¹¹¹In coupled to monoclonal antibodies with 1-(P-isothiocyanatobenzyl)-DPTA has shown little uptake in non-tumorous tissues, particularly the liver, and therefore enhances specificity of tumor localization (Esteban et al., J. Nucl. Med. 28:861-870 (1987)).

Examples of suitable non-radioactive isotopic labels include ¹⁵⁷Gd, ⁵⁵Mn, ¹⁶²Dy, ⁵²Tr, and ⁵⁶Fe.

Examples of suitable fluorescent labels include an ¹⁵²Eu label, a fluorescein label, an isothiocyanate label, a rhodamine label, a phycoerythrin label, a phycocyanin label, an allophycocyanin label, an o-phthaldehyde label, and a fluorescamine label.

Examples of suitable toxin labels include diphtheria toxin, ricin, and cholera toxin.

Examples of chemiluminescent labels include a luminal label, an isoluminal label, an aromatic acridinium ester label, an imidazole label, an acridinium salt label, an oxalate ester label, a luciferin label, a luciferase label, and an aequorin label.

Examples of nuclear magnetic resonance contrasting agents include heavy metal nuclei such as Gd, Mn, and iron.

Typical techniques for binding the above-described labels to antibodies are provided by Kennedy et al., Clin. Chim. Acta 70:1-31 (1976), and Schurs et al., Clin. Chim. Acta 81:1-40 (1977). Coupling techniques mentioned in the latter are the glutaraldehyde method, the periodate method, the dimaleimide method, the m-maleimidobenzyl-N-hydroxy-succinimide ester method, all of which methods are incorporated by reference herein.

### (C) Screening for Serotype k S. mutans Strains

The present invention provides a method of screening for serotype *k S. mutans* strains from a biological sample. In one aspect, the present invention provides a method of screening for strains of a serotype that has a reduced amount of glucose side chain in the serotype-specific polysaccharide antigen of *S. mutans,* using a polynucleotide, or a fragment thereof, that comprises a base sequence, or a complementary sequence thereof, which includes the base sequence of *any of* SEQ ID NO: 1 through 4, and which is specific to the serotype strains with a reduced amount of glucose side chain in the serotype-specific polysaccharide antigen of *S. mutans.* In a preferred embodiment, the serotype strains with a reduced amount of glucose side chain in the serotype-specific polysaccharide antigen of *S. mutans* are *S. mutans* strains of serotype *k*. Further, in a preferred embodiment, a screening method of the present invention includes the step of determining a serotype of *S. mutans* strains with use of antibodies of the present invention.

In another aspect, the present invention provides a method of screening for serotype strains that have a reduced amount of glucose side chain in the serotype-specific polysaccharide antigen of *S. mutans,* using antibodies that specifically bind to the serotype strains with a reduced amount of glucose side chain in the serotype-specific polysaccharide antigen of *S. mutans.* In a preferred embodiment, the serotype strains with a reduced amount of glucose side chain in the serotype-specific polysaccharide antigen of *S. mutans* are *S. mutans* strains of serotype *k*. Further, in a preferred embodiment, a screening method of the present invention includes the step of determining a serotype of *S. mutans* strains with use of a polynucleotide, or a fragment thereof, of the present invention.

Further, the present invention provides strains of a serotype with a reduced amount of glucose side chain in the serotype-specific polysaccharide antigen of *S. mutans,* as identified by a screening method of the present invention. In a preferred embodiment, the strains of a serotype with a reduced amount of glucose side chain in the serotype-specific polysaccharide antigen of *S. mutans* are *S. mutans* strains of serotype *k.* Further, in a preferred embodiment, the serotype *k S. mutans* strains are, but not limited to, TW295, TW871, FT1 through FT51, SU1 through SU51, YK1 through YK50, FT1GD, YK1R, AT1, and YT1.

### (3) Detection Kit and Diagnosis Kit for Serotype k S. mutans Strains

A *S. mutans* serotype determining kit according to the present invention is for determining whether tested *S. mutans* strains are serotype *k.*

The present invention provides a kit for detecting serotype *k S. mutans* strains, wherein the kit includes an oligonucleotide of the base sequence, or a complementary sequence thereof, of at least 12 contiguous bases in the base sequence of *any of* SEQ ID NO: 1 through 4. Preferably, a kit of the present invention further includes an oligonucleotide that specifically binds to *S. mutans* strains of serotype *c*, *e*, or *f*. More preferably, the oligonucleotide is labeled. With the present invention, a patient carrying serotype *k S. mutans* strains can readily be identified. More generally, the invention enables detection of serotype *k S. mutans* strains in cases which exceeded the detection limit.

In one aspect, the present invention provides a kit in which the oligonucleotide of the invention is used as a PCR primer. In a preferred embodiment, a kit of the present invention further includes a reagent to be used in PCR reactions.

In another aspect, the present invention provides a kit in which the oligonucleotide of the invention is used as a hybridization probe. In a preferred embodiment, a kit of the present invention further includes a reagent to be used in hybridization reactions.

In one embodiment of the invention, a kit of the present invention includes an oligonucleotide of the base sequence of SEQ ID NO: 9. Preferably, a kit of the present invention includes an oligonucleotide of the base sequence of SEQ ID NO: 8. More preferably, a kit of the present invention includes an oligonucleotide of the base sequence of SEQ ID NO: 10.

Further, the invention provides a kit for detecting *S. mutans* strains of serotype *k,* wherein the kit includes antibodies that specifically bind to the polysaccharide antigen of serotype *k S. mutans* strains. Preferably, the oligonucleotide is labeled. With the present invention, a patient carrying serotype *k S. mutans* strains can readily be identified. More generally, the invention enables detection of serotype *k S. mutans* strains in cases which exceeded the detection limit.

### (4) Detection Tools and Diagnostic Tools for Serotype k S. mutans Strains

A bacteria detection tool according to the present invention is for detecting and identifying bacteria in a sample, wherein bacteria in a sample are detected and identified through hybridization of sample-derived nucleic acids with oligonucleotides that are immobilized on a substrate surface and are based on the base sequences that are specific to the species or genus to which the tested bacteria belong. When used for the detection of serotype *k S. mutans* strains, the oligonucleotide include a base sequence, or a complementary sequence thereof, of at least 12 contiguous bases of the base sequence of the polynucleotide that encodes an enzyme associated with the biosynthesis of the polysaccharide antigen specific to serotype *k S. mutans* strains. When comprehensively testing other species of bacteria, it is preferable that oligonucleotides that include base sequences specific to one or more species of those bacteria be also immobilized on the substrate. By using the kit in an assay that uses a sample including more than one species of bacteria, it is possible to obtain information as to which bacterial species are more likely to coexist with serotype *k S. mutans* strains.

### (A) Substrate

The material of the substrate used in a bacteria detection tool of the present invention is not particularly limited as long as it can stably immobilize the oligonucleotides. Non-limiting examples include synthetic resin such as polycarbonate or plastic, and glass. The shape of substrate is not particularly limited either. For example, the substrate is preferably in the form of a plate or a film.

### [Oligonucleotides Immobilized on a Substrate Surface]

The oligonucleotides immobilized on a surface of the substrate of the bacteria detection tool of the present invention are based on base sequences specific to the species or genus to which the tested bacteria belong. By establishing hybridization between the oligonucleotides and the nucleic acids derived from the sample, it is possible to detect the bacteria of the target species or genus included in the sample. In the following, the term "capture oligonucleotides" may be used to refer to oligonucleotides that are based on base sequences specific to the species or genus to which the tested bacteria belong.

The specific base sequences may be species- or genus-specific base sequences in the genome of tested bacteria. However, the specific base sequences are more preferably base sequences that correspond to the ribosomal RNA gene of tested bacteria. Among such ribosomal RNA genes, 16S ribosomal RNA gene of bacteria is known to include many species- or genus-specific base sequences, and therefore it is particularly preferable that the species- or genus-specific base sequences be found in the DNA sequence that corresponds to 16S ribosomal RNA gene. Base sequences of ribosomal RNA genes are available from GenBank, EMBL, DDBJ, or other databases.

The capture oligonucleotides are designed based on the genus- or species-specific base sequences. As such, the capture oligonucleotides may be the genus- or species-specific base sequences themselves, or may include mutation, provided that the specific hybridization with the nucleic acids prepared from the tested bacteria is established. The position of the mutation is not particularly limited.

The length of the capture oligonucleotides (the number of bases) is not particularly limited. However, detection of hybridization becomes difficult when it is too short, whereas the oligonucleotide allows for undesirable non-specific hybridization when it is too long. The inventors of the present invention worked to find an optimal length of the capture oligonucleotides, and determined the standard length to be 12 to 50 base long. Preferably the capture oligonucleotide is 12 to 40 base long, more preferably 12 to 30 base long, and even more preferably 13 to 22 base long. However, the length of the capture oligonucleotide is not limited to these examples. The length of the base sequence is dependent on the sequence characteristics (the content of specific bases, the number of repeats of the same base). In fact, the inventors of the present invention have confirmed that even a short sequence is capable of specific hybridization if it provides good adhesion.

In the case where the capture oligonucleotide has a hairpin structure, a loop structure, or any other conformation that hinders the hybridization with the nucleic acids derived from the sample, one or more nucleotides of the capture oligonucleotide are replaced with nucleic acids that do not pair up with inosine or other nucleotides. In this way, such conformation of the capture oligonucleotide can be cancelled.

The method of synthesis of the capture oligonucleotides is not particularly limited, and the capture oligonucleotides can be synthesized by a known method as described in, for example, Maniatis, T. et al., Molecular Cloning A Laboratory Manual, Second Edition, Cold Spring Harbor Laboratory Press (1989). Generally, the oligonucleotides are synthesized with a commercially available DNA synthesizer.

In a bacteria detection tool according to the present invention, it is preferable that, in addition to the oligonucleotides that are based on the base sequences specific to the species or genus to which the tested bacteria belong, another type of oligonucleotide, known as the control capture oligonucleotide, be immobilized on a surface of the substrate. The control capture oligonucleotide has positive and negative forms. The positive control capture oligonucleotide is used to check the progress of the amplification reaction in a probe preparing step to be described later. The negative control capture oligonucleotide is used to confirm that there is no non-specific hybridization or pseudo-positive hybridization signals. The present invention also includes a bacteria detection tool in which such positive and negative control capture oligonucleotides are immobilized on a substrate surface.

The positive control capture oligonucleotide is not particularly limited as long as it is designed based on the base sequence included in the probe prepared from the tested bacteria. In the case where more than one type of bacteria is simultaneously detected with the same bacteria detection tool, the positive control capture oligonucleotide may be designed for each type of tested bacteria, or may be designed based on a common base sequence of the probes prepared from these bacteria. When the probes prepared from the bacteria do not have a common base sequence, the positive control capture oligonucleotide may be designed for each different group of bacteria. Alternatively, the positive control oligonucleotide may be a portion of an artificially designed sequence that includes the same primer sequence but are different from the sequence of the tested bacteria. By using such an artificial sequence as a template, a probe is prepared and supplemented to the probe prepared from the sample (such a probe will be referred to as a control probe). This allows for testing of specificity of hybridization. Details of the probe will be described later.

Preferably, the negative control capture oligonucleotide is designed to include the base sequence of the positive control capture oligonucleotide with the intentionally induced base substitution in which one or more bases are replaced and in which the number of bases replaced does not exceed 20% of the total number of bases in the base sequence of the positive control capture oligonucleotide. The number of bases to be replaced is determined in consultation with the hybridization conditions, and it is decided so as not to cause hybridization with the probes derived from the tested bacteria.

The type of bacteria to be detected is not particularly limited, and any bacteria to be detected from a sample of interest can be suitably selected. For example, bacteria that have the potential risk of entering and contaminating food can be detected. Entry of toxic bacteria into food poses a serious hazard to public health. The presence and/or amount of serotype *k S. mutans* strains can be measured by comparing these parameters with those of food contaminating bacteria.

Examples of such food contaminating bacteria include *Lactobacillus, Pediocuccus, Streptococcus, Megasphaera,* and *Pectinatus.* Examples of bacterial species that belong to *Lactobacillus* are *Lactobacillus brevis, Lactobacillus coryniformis, Lactobacillus curvatus, Lactobacillus delbrueckii, Lactobacillus fermentum, Lactobacillus lindneri, Lactobacillus malefermentans, Lactobacillus casei, Lactobacillus rhamnosus, Lactobacillus buchneri, Leuconostoc* and *Zymomonas* bacteria, *Enterococcus durans,* and *Lactococcus lactis.* However, the type of bacteria to be detected is not just limited to these examples.

The capture oligonucleotide for detecting and identifying the bacteria may be an oligonucleotide of a genus-specific base sequence within the base sequence of the 16S ribosomal RNA gene of the bacteria. However, the capture oligonucleotide is not just limited to this example.

The capture oligonucleotide immobilized on a substrate surface of a bacteria detecting tool of the present invention is not particularly limited as long as it can hybridize with the probe prepared from the tested bacteria.

One or more kinds of capture oligonucleotides may be immobilized on a single substrate. For the detection of bacteria contained in a sample, it is generally preferable, for ease of handling and test speed, that the bacteria in a sample be detected with a single substrate. As such, it is most preferable that a bacteria detecting tool according to the present invention be a tool known as a micro array, in which a plurality of capture oligonucleotides corresponding to the species or genus of the target bacteria are immobilized on a single substrate.

### (B) Immobilization of Oligonucleotide (Capture Oligonucleotide)

A method by which the oligonucleotide is immobilized on a substrate surface is not particularly limited, and conventional methods can be suitably used. For example, techniques used in a common hybridization method can be used, including, for example, physical absorption, electron bonding, and covalent bonding. In a bacteria detecting tool according to the present invention, oligonucleotides are preferably immobilized on a substrate having carbodiimide groups or isocyanate groups on its surface (USP 5,908,746, Japanese Laid-Open Patent Publication No. 8-23975).

In spotting the oligonucleotides, the reactivity between oligonucleotides and probes may not be sufficient when the amount of spot is too small. In this case, accurate results may not be obtained. As for high-density spotting, the technique is associated with various technical problems and costly. Further, it requires a more precise and expensive detector (e.g., scanner) for the detection of hybridization signals that involve fluorescent labeling or chemical coloring of the probes. It is therefore preferable that the oligonucleotides be immobilized in 10 µm to 1000 µm on a substrate surface. A method by which the oligonucleotides are immobilized on the substrate is not particularly limited. For example, the oligonucleotides may be immobilized by spotting a solution of oligonucleotide on the substrate, using a spotting machine. In this case, the oligonucleotide solution can be spotted usually in circles.

### (5) Use of the Present Invention

The present invention provides convenient ways to determine whether a patient is a carrier of bacterial strains that are likely to be the causative strains of *S. mutans,* one of the causative bacteria of IE. The invention therefore enables causative bacteria to be detected in cases which conventionally exceeded the detection limit. This narrows down the number of individuals carrying the bacterial strains having a high risk of causing infective endocarditis. As a result, the amount of antibiotics can be reduced and emergence of resistant bacteria due to use of antibiotics can be suppressed. Further, correlation can be found between the causative *S. mutans* strains and symptoms, in addition to providing information regarding relations with other causative bacteria.

The following will describe the present invention in more detail by way of Examples. It should be noted that the invention is not limited by the following description.

### [Example 1]

### (A) Collection of S. mutans Clinical Oral Isolates and Identification of S. mutans serotypes

One thousand three hundred twenty-six *S. mutans* strains isolated from 571 children between 1982 and 1990 (MT4000 or MT10000 series of isolates) were selected from the laboratory stock of the inventors.

In addition, strains from 100 subjects (3 to 17 years of age; average 8.9 years old) who visited the Pedodontics Clinic of Osaka University Dental Hospital, Suita, Osaka, Japan, in August in 2002 were randomly selected (the NN2000 series of isolates). These subjects included 88 healthy children along with 5 patients with cleft lip and palate, 3 with ventricular septal defect, 2 with amelogenesis imperfecta, 1 with spondyloschisis, and 1 autistic patient.

Collection of clinical specimens was carried out in accordance with the Osaka University Health Guideline for Studies Involving Human Subjects. Plaque samples were collected in sterile PBS, diluted and streaked onto MS agar (Difco) plates containing bacitracin (0.2 units/ml; Sigma Chemical Co., St. Louis, Mo.) and 15% (w/v) sucrose. One colony from each subject was chosen on the basis of colonial morphology and used for experiments.

Serotyping was determined by the immunodiffusion method as described in Masuda, N. et al., 1985. Transmission of Streptococcus mutans in some selected families. Microbios. 44: 223-232, using rabbit antisera specific for serotypes *c*, *e*, and *f*.

It was found that all of the MT series 1326 strains were serologically classified as *c*, *e*, or *f*, and with no untypable isolates found. On the other hand, 78, 17, and 3 of the 100 isolates (NN 2000 series) were classified as serotype *c*, *e*, and *f*, respectively, whereas the remaining 2 strains, FT1 (NN2011) and SU1 (NN2029), were found to be not react with *c*, *e*, or *f* specific antiserum (Table 1). In addition, 50 colonies were isolated from each subject with a serologically untypable strain (FT1 or SU1).

**(Table 1. Serotype distribution in 2 series of clinical oral isolates of S. mutans)**

| Serotype | MT series taken between 1982 and 1990 (N=1326) | NN2000 series taken in 2002 (N=100) |
|---|---|---|
| *c* | 1131 | 78 |
| *e* | 163 | 17 |
| *f* | 32 | 3 |
| *k* | 0 | 2 |

### (B) S. mutans Strains

With the antisera to the type *c, e,* or *f S.* mutans strains, the blood isolates TW295 and TW871 were identified as serologically untypable strains, whereas the blood isolates TW964 and TW1378 were *f* type and *e* type strains, respectively. Table 2 shows properties of these *S. mutans* strains and blood isolates MT8148 (*c* type strain), MT4245 (*e* type strain), and MT4251 (*f* type strain).

**(Table 2. Bacterial strains used in the present example)**

| Strain | Serotype | Features |
|---|---|---|
| TW295 | *k* | Blood isolate from a 59-year-old male with bacteremia following a tooth extraction procedure |
| TW871 | *k* | Blood isolate from a 45-year-old female with infective endocarditis complicated with subarachnoid hemorrhage |
| TW964 | *f* | Blood isolate from a 72-year-old male with Infective endocarditis |
| TW1378 | *e* | Blood isolate from a 59-year-old male with infective endocarditis |
| MT8148 | *c* | Oral isolate from a healthy child |
| MT4245 | *e* | Oral isolate from a healthy child |
| MT4251 | *f* | Oral isolate from a healthy child |
| NN2001 | *c* | Oral isolate from a 6-year-old healthy girl |
| NN2002 | *e* | Oral isolate from a 9-year-old healthy boy |
| NN2003 | *f* | Oral isolate from a 7-year-old healthy boy |
| MT8148GD | *k* | Glucose side chain defective mutant strain of MT8148 |
| FT1(NN2011) | *k* | Oral isolate from a 3-year-old healthy girl |
| SU1(NN2029) | *k* | Oral isolate from a 10 year-old healthy girl |
| YK1 | *k* | Oral isolate from a 6-year-old girl with Down's syndrome |

### (C) Generation of Antisera against S. mutans

Antisera to untypable *S. mutans* strains were obtained. Due to a considerably reduced level of antigenicity, antisera to the *S. mutans* strains of a novel serotype could not be obtained by conventional methods. The inventors of the present invention modified the method of immunization and successfully obtained such antisera.

Specifically, whole cells of each of TW295 and TW871 strains were suspended in phosphate-buffered saline (PBS), and injected intravenously into the auricular vein of rabbits repeatedly for 5 consecutive days (dry weight; 5 mg per day). One week after the final injection, immunization was repeated another 2 weeks, 5 times each week. Blood was then taken from the auricular vein and the antisera to the TW295 and TW871 strains were obtained.

### (D) Construction of Glucose Side Chain Defective Mutant Strain, and Microbial and Serological Characterization

A glucose side chain defective (GD) mutant strain of MT8148 was constructed by insertional inactivation of the *gluA* gene with an antibiotic-resistant gene. The *gluA* gene and its flanking region were amplified by polymerase chain reaction (PCR) using AmpliTaq Gold polymerase (Applied Biosystems, Foster City, Calif.), with primers constructed on the basis of the *gluA* sequence from *S. mutans* strain Xc (Genbank accession no. AB001562) and the *S. mutans* genomic database published by Oklahoma University (Genbank accession no. AE014133). The amplified fragment was then cloned into a pST Blue-1 vector (Promega, Madison, Wis.) to generate plasmid pRN 101. The middle of the open reading frame of *gluA* in pRN101 was cleaved by *Stu* I, then ligated with the erythromycin resistant gene *(erm)* from recombinant plasmid pKN100 carrying an 830 bp fragment of erm from pVA838 to yield pRN102. After linearization by digestion with restriction enzyme *Not* I, the plasmid was introduced into *S. mutans* MT8148 by the method of Tobian and Macrina. The transformants were screened on Mitis-salivarius (MS) agar (Difco Laboratories, Detroit, Mich.) plates containing erythromycin (10 µg/ml). The appropriate insertional inactivation of the mutant MT8148GD was confirmed by PCR amplification of the *gluA* gene and immunodiffusion method.

A *gluA*-inactivated mutant strain, MT8148GD, showed typical biological features of *S. mutans* including rough colonies on MS agar, positive profiles of sugar fermentation, expressions of GTFs and PAc, and high levels of sucrose-dependent adhesion and cellular hydrophobicity.

### (E) Reactivity of Antisera to Various S. mutans Strains

In order to perform an immunodiffusion method (gel precipitation reaction) with the antibodies, the TW295 and TW871 strains and the type *c, e,* and *f S. mutans* strains were incubated overnight. After 15 minute heating at 121°C, the samples were centrifuged and antigens (Rantz-Randall (RR)) were obtained in the supernatant.

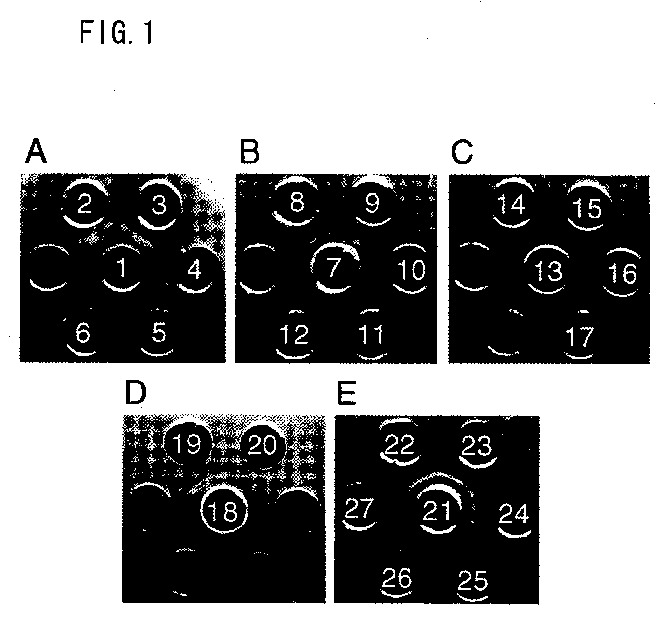

The antisera to TW295 and TW871 *S. mutans* strains did not react with the antigens from the *c, e,* and *f* type *S. mutans* strains, but reacted with the antigens from the TW295 and TW871 strains (Figs. 1A through 1E). The serotype of TW295 and TW871 strains was designated as serotype *k*.

The center well shown in Fig. 1A contained RR extract from strain TW295 (well 1). The outer wells contained antisera to strain TW295 (well 2), and TW871 (well 3), along with antisera specific to serotypes *c* (well 4), *e* (well 5), and *f* (well 6). The center well shown in Fig. 1B contained antiserum to strain TW871 (well 7). The outer wells contained RR extracts from strains TW295 (well 8), TW871 (well 9), MT4251 (*f*) (well 10), MT4245 (*e*) (well 11), and MT8148 (*c*) (well 12). The center well shown in Fig. 1C contained RR extract from strain FT1 (well 13). The outer wells contained antisera specific to strain TW871 (well 14), serotype *c* (well 15), serotype *e* (well 16), and serotype *f* (well 17). The center well shown in Fig. 1D contained antiserum specific to serotype *c* (well 18). The outer wells contained RR extracts from strains MT8148 (well 19), and MT8148GD (well 20). The center well shown in Fig. 1 E contained antiserum to strain TW871 (well 21). The outer wells contained RR extracts from strains TW295 (well 22), TW871 (well 23), FT1 (well 24), SU1 (well 25), YK1 (well 26), and MT8148GD (well 27).

The RR extract of MT8148GD produced a precipitation band with the antiserum to TW871, however, not with serotype *c* specific antiserum (Fig. 1D), and fused to precipitation bands of TW295, TW871, FT1, SU1, and YK1.

The antisera were used to examine frequencies of type *k* strains in the *S. mutans* of 100 isolates from 100 subjects (NN2000 series). It was demonstrated that the RR antigens of 2 of 100 isolates were not reactive with anti-*c*, *e,* and *f,* though they were reactive with anti-*k* antisera. The RR antigens of the remaining 98 isolates were reactive to any of the type *c, e,* and *f* specific antisera. From these results, the untypable strains (FT1 and SU1) of the clinical oral isolates of *S. mutans* from NN2000 series were identified as serotype *k*.

### (F) Characterization of Untypable S. mutans

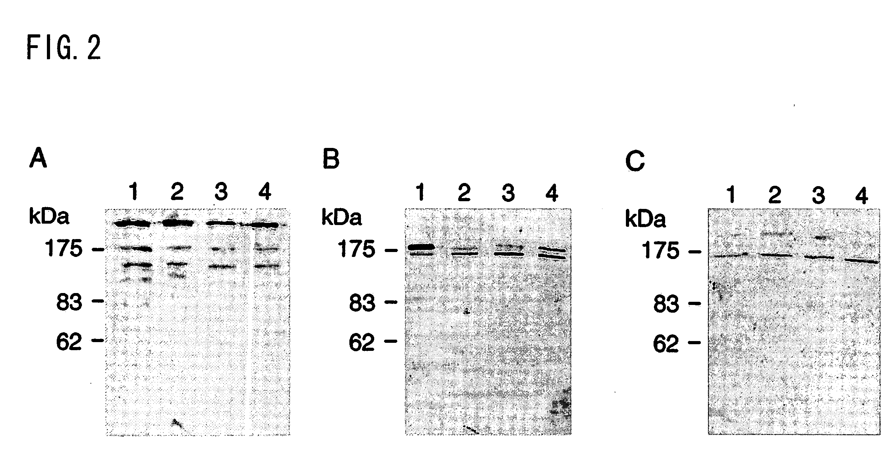

Sucrose-dependent adhesion and cellular hydrophobicity of the untypable clinical isolates were evaluated as described by Hamada et al., and Rosenberg et al., respectively. The expressions of cell-associated (CA-) or cell-free (CF-) glucosyltransferases (GTFs) and the surface protein antigen c (PAc) were analyzed using Western blot analysis with antibodies specific to GTF and PA (Figs. 2A through 2C). Genomic DNA was extracted from the test organisms, and 16S rRNA was sequenced and compared with that of reference strain NCTC 10449 (GenBank accession no. X58303, S70358).

Referring to Figs. 2A and 2B, whole cells were separated by 7% SDS polyacrylamide gel electrophoresis and reacted with rabbit antibodies against PAc (Fig. 2A) or CA-GTF (Fig. 2B) of *S. mutans.* In Fig. 2C, the transferred protein bands on a PVDF membrane of culture supernatant concentrated by ammonium sulfate precipitation (C) were separated by 7% SDS polyacrylamide gel electrophoresis and reacted with rabbit antibodies against CF-GTF (C). Lane 1, strain MT8148; lane 2, strain FT1; lane 3, strain SU1; lane 4, strain YK1. Strains FT1 and SU1 expressed PAc and three types of GTFs.

Strains FT1 and SU1 showed high levels of sucrose-dependent adhesion and cellular hydrophobicity (data not shown), and their 16S rRNA gene sequences were identical to that of strain NCTC 10449 (Genbank accession no. X58303 and S70538). In addition, the RR extracts of strains FT1 produced a precipitation band with antisera to strain TW871 and TW295, which also fused to a precipitation band of the RR extract of TW871 with antiserum to TW871 (Fig. 1E). Furthermore, fifty strains from each subject with strain FT1 or SU1 were not reactive with *c*, *e*, or *f* specific antiserum.

Further, strains FT1 and SU1 both showed rough colonies on MS agar plates, positive bacitracin resistance, gamma hemolysis on blood agar, a positive fermentation profile for mannitol, sorbitol, raffinose, and melibiose, and a negative dextran agglutination, which are characteristics of *S. mutans.*

### (G) Further Analysis of Clinical Isolates

Two thousand five hundred clinical isolates of *S. mutans* were obtained from another group of 50 subjects (3 to 19 years of age; average 7.8 years old) who came to the Pedodontics Clinic of Osaka University Dental Hospital in early 2003. These included 45 healthy children and 5 patients with general or oral health problems such as Down's syndrome, congenital heart disorder, cleft lip and palate, amelogenesis imperfecta, and oligodontia.

Table 3 shows the serotype distribution of 2500 recent isolates of *S. mutans,* of which 2450 were classified as *c*, *e*, or *f* type.

**(Table 3. Serotype distribution of 2500 S. mutans isolates obtained from 50 subjects)**

| Serotype | Number of isolates | Number of subjects |
|---|---|---|
| *c* | 1769 | 38 |
| *e* | 551 | 13 |
| *f* | 130 | 4 |
| *k* | 50 | 1 |

On the other hand, the RR extracts of the other 50 strains (YK1 through YK50) from a single subject (a girl with Down's syndrome) produced a precipitation band with antiserum to TW871, which also fused to the precipitation band of RR extracts of TW295, TW871, FT1, and SU1. Table 4 shows the serotype distribution patterns in individual subjects, most of whom were found to possess serotype *c* of *S. mutans* alone, followed by serotype *e* alone, while 5 of the 50 subjects possessed multiple serotypes.

**(Table 4. Individual serotype distribution among 50 subjects)**

| Serotype | Number of subjects |
|---|---|
| (Single serotype) | |
| *c* | 34 |
| *e* | 8 |
| *f* | 2 |
| *k* | 1 |

| (Multiple serotype) | |
|---|---|
| *c* and *e* | 3 |
| *e* and *f* | 1 |
| *c*, *e*, and f | 1 |

### (H) Phagocytosis Assay

Organisms were cultured in Brain Heart Infusion broth (Difco) for 18 h at 37°C. After washing the bacterial cells, cell concentrations were adjusted with PBS to 1.0 x 10⁸ CFU/ml. Human peripheral blood (500 ml) was collected from a healthy volunteer and incubated with 500 ml (5.0 x 10⁷ CFU) of the tested bacteria for 10 min at 37°C. Interactions between polymorphonuclear leukocytes (PMNs) and bacteria were observed following Giemsa staining (Wako Pure Chemical Industries, Osaka, Japan) and a light microscope (magnification, x100; Olympus Optical, Tokyo, Japan). The rate was expressed by the mean ratio of phagocytosed PMNs per 100 PMNs, with 500 PMNs examined.

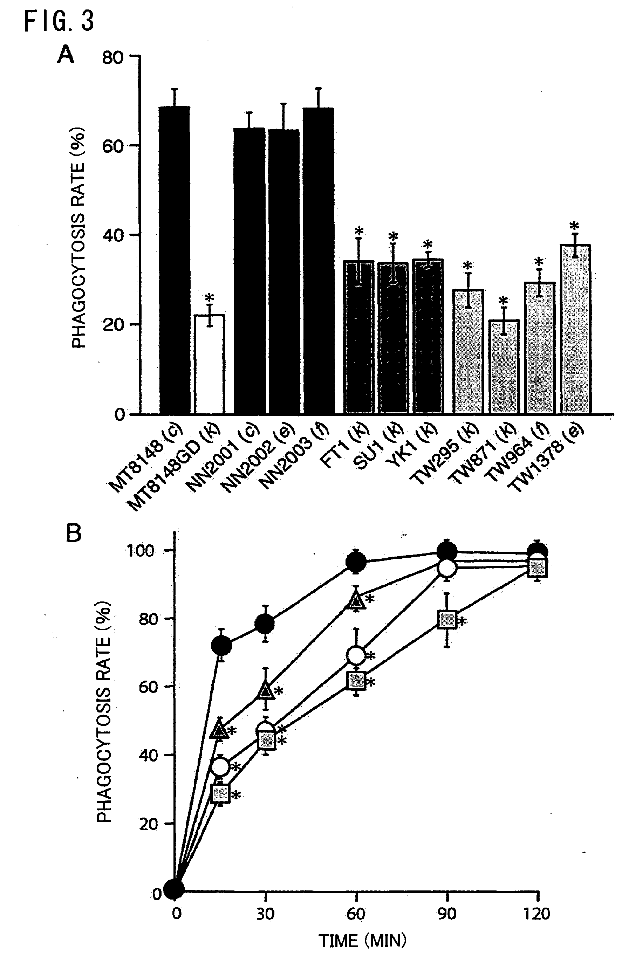

In addition, changes in the rate of phagocytosis in strains MT8148, MT8148GD, FT1, and TW295 were observed at 15, 30, 60, 90, and 120 min. Intergroup differences of various factors were estimated by a statistical analysis of variance (ANOVA) for factorial models (Fig. 3).

Figs. 3A and 3B show phagocytosis rate of oral and blood isolates of *S. mutans.* The results show the mean±standard deviation from 5 experiments. In Fig. 3A, the phagocytosis rate of the 12 strains was examined following 10 min of incubation. In Fig. 3B, changes in the phagocytosis rate of strains MT8148 (solid circle), MT8148GD (blank circle), FT1 (solid triangle), and TW295 (blank triangle) at 15, 30, 60, 90, and 120 min of incubation.

The phagocytosis rate of strain MT8148GD was 22.0±2.4%, which was significantly lower than the parent strain MT8148 (68.4±4.1%) (*P*<0.001). Further, oral isolates of NN2001 (*c*), NN2002 (*e*), and NN2003 (*f*) showed a similar phagocytosis rate to MT8148, while the serotype *k* oral isolate strains FT1, SU1, and YK1 also showed a significantly lower phagocytosis rate than MT8148 (P<0.001). In addition, the phagocytosis rates of all 4 blood isolate strains were significantly lower than those of the oral isolates (*P*<0.001), while that of MT8148GD, FT1, and TW295 was significantly lower than MT8148 until 60 min of incubation (*P*<0.001), and that of TW295 was also significantly lower than MT8148 after 90 min of incubation (*P*<0.001) (Fig. 3B).

IE is known to be initiated by an invasion of pathogenic bacteria into the bloodstream, however, the mechanisms of invasion and survival of *S. mutans* in blood have not yet been clarified. In the present Example, the inventors found that the phagocytosis rate of the glucose side chain defective isogenic mutant strain was lower than that of the parent strain. There were statistically significant differences between MT8148 (serotype c) and the other strains, as determined by Fisher's PLSD analysis (* *P*<0.001).

In addition, the serotype *k* oral and blood isolates had lowered PMN phagocytosis rates. Together, these findings suggest that serotype *k* strains, which are considered to lack a glucose side chain in serotype specific polysaccharide, may be possible bacteremia pathogenic strains. On the other hand, the blood isolate strains TW964 (*e*) and TW1378 (*f*) were less susceptible to phagocytosis than the oral isolates NN2002 (e) and NN2003 (*f*), though we have shown that TW964 lacks a glucan-binding protein A, whereas any such alteration of TW1378 has not been found. Thus, there may be an alteration of cell surface structures other than the serotype specific polysaccharide that has an effect on phagocytic ability.

### (I) Discussion

The RR extracts of 2 blood isolates (TW295 and TW871), 152 oral isolates (FT1 through FT51, SU1 through SU51, and YK1 through YK50), and a *gluA*-inactivated strain (MT8148GD) produced precipitation bands with antisera to TW295 or TW871. These isolates were shown to possess biological properties typical of *S. mutans,* including high levels of sucrose-dependent adhesion and hydrophobicity, and glucosyltransferases.

The isolation frequencies of *S. mutans* serotypes *c, e,* and *f* in the present study were closely similar to those reported in a Denmark study. In that report, 1 of 76 mutans streptococci from human dental plaque samples and 7 of 70 from human carious lesions, could not be serologically classified as serotype *a* through *g.* A biochemical analysis revealed that these untypable strains belonged to *S. mutans,* while there was no description of the structures of the serotype specific polysaccharide. In another study, all 1047 *S. mutans* or *S. sorbinus* isolates taken from human dental plaque, carious dentin, or feces samples were able to be classified as serotype *c*, *e*, *f, d*, or *g*. Further, in a recent study conducted in another area of Japan, 144 strains of *S. mutans* or *S. sorbinus* from dental plaque were classified as serotype *c*, *e*, *d*, or *g*, and there were no serotype *f* strains or untypable strains detected. On the other hand, 9 out of 103 subjects were reported to harbor serologically untypable *S. mutans* in Japan recently, although precise description of the properties of the serotype specific polysaccharide was not described. Taken together, we speculated that serotype *k* organisms have appeared in Japan only recently.

In the present study, *S. mutans* strains with this new serotype were found in plaque samples from 3 children, with a total of 152 classified as serotype *k*. The origin of these clinical isolates has not yet been identified, however, serotype *k* strains occupied the majority of the *S. mutans* strains found in dental plaque samples from those 3 subjects, and all showed high levels of cellular hydrophobicity and sucrose-dependent adherence, as well as lower rates of phagocytosis. These findings suggest that serotype *k* strains of *S. mutans* are present in the oral cavity of humans, because of their high hydrophobicity and sucrose-dependent adhesion, along with the expressions of GTFs and PAc, and may be able to survive in blood due to their lower phagocytotic capability. Therefore, special care must be taken for children with heart disorders. In particular, it may be more risky for Down's syndrome patients with *S. mutans* serotype *k* strain in the oral cavity, as they have been reported to have a possible congenital basis for disorders of PMN functions, and are also known to be susceptible to ventricular septal defect. As detection of new serotype *k* in these patients may indicate an increased risk, a clinical approach such as antibiotics prescription prior to dental treatment may be required and should be kept in mind.

### [Example 2: Search for Gene Sequences Specific to Serotype k S. mutans Strains]

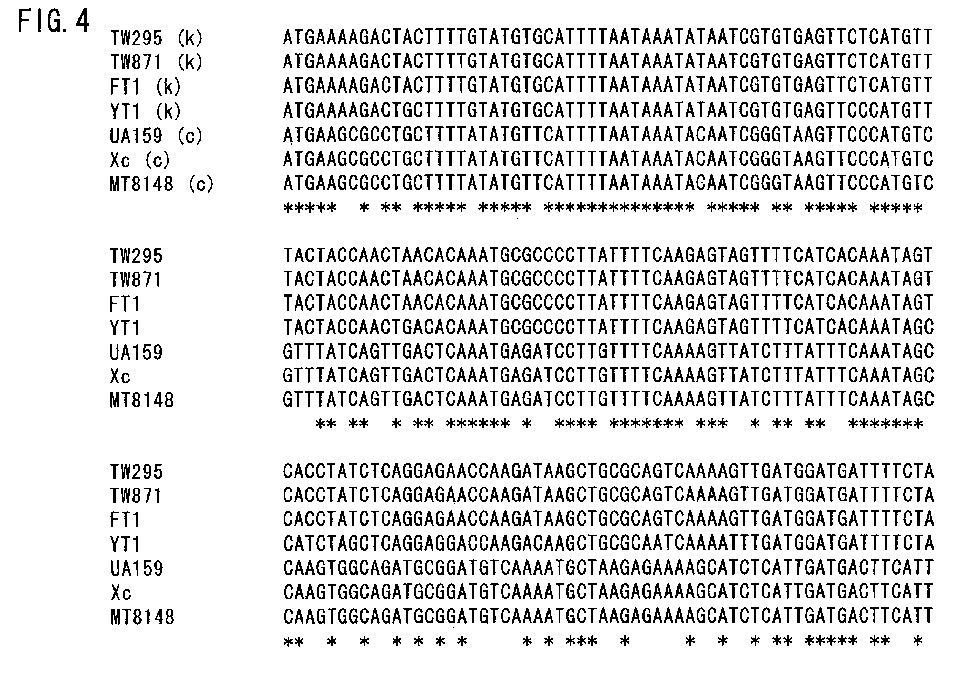

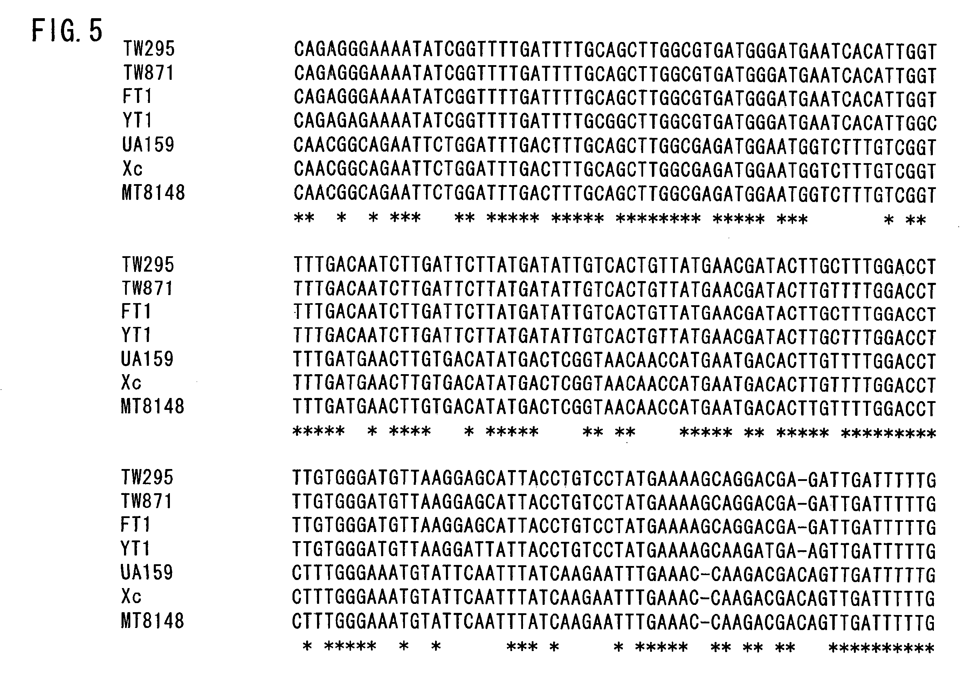

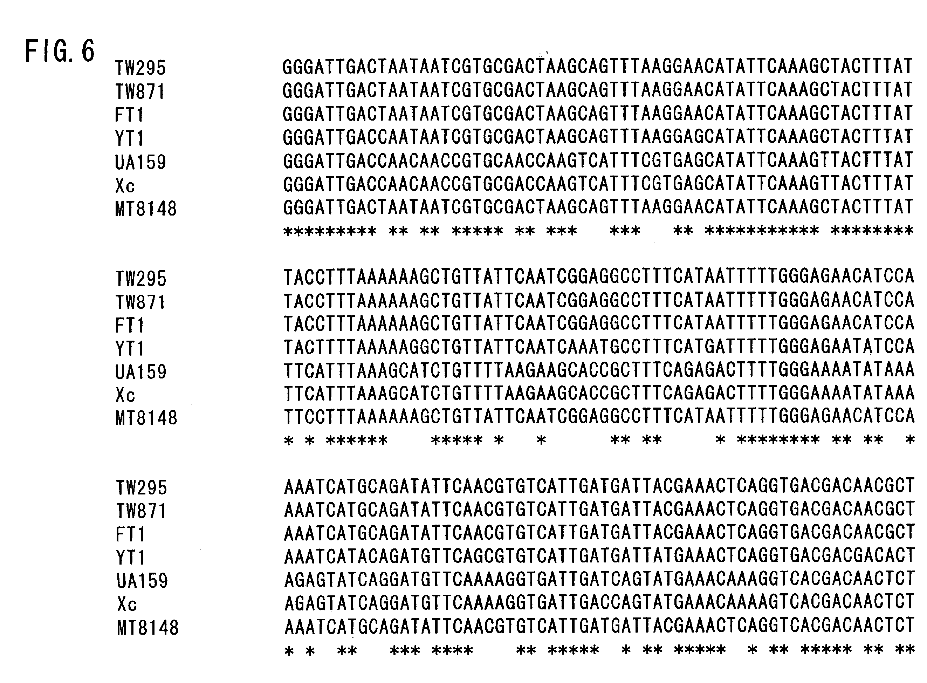

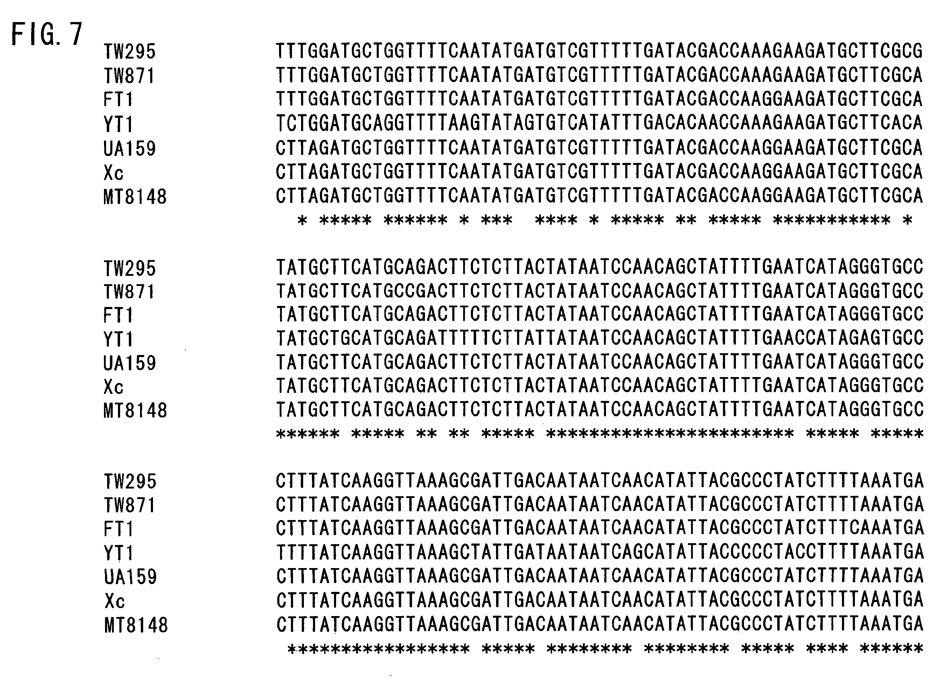

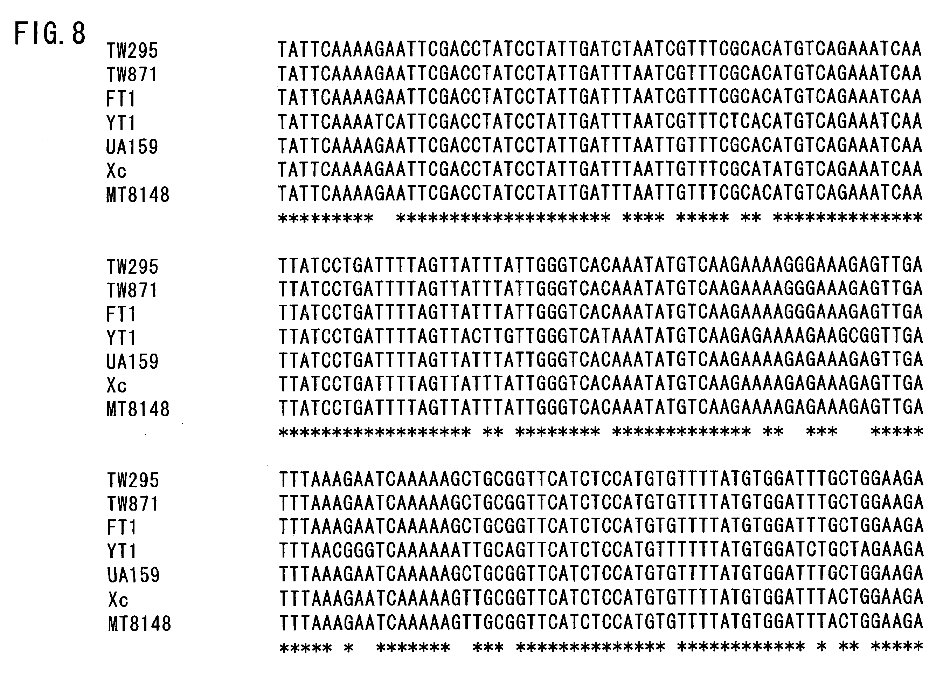

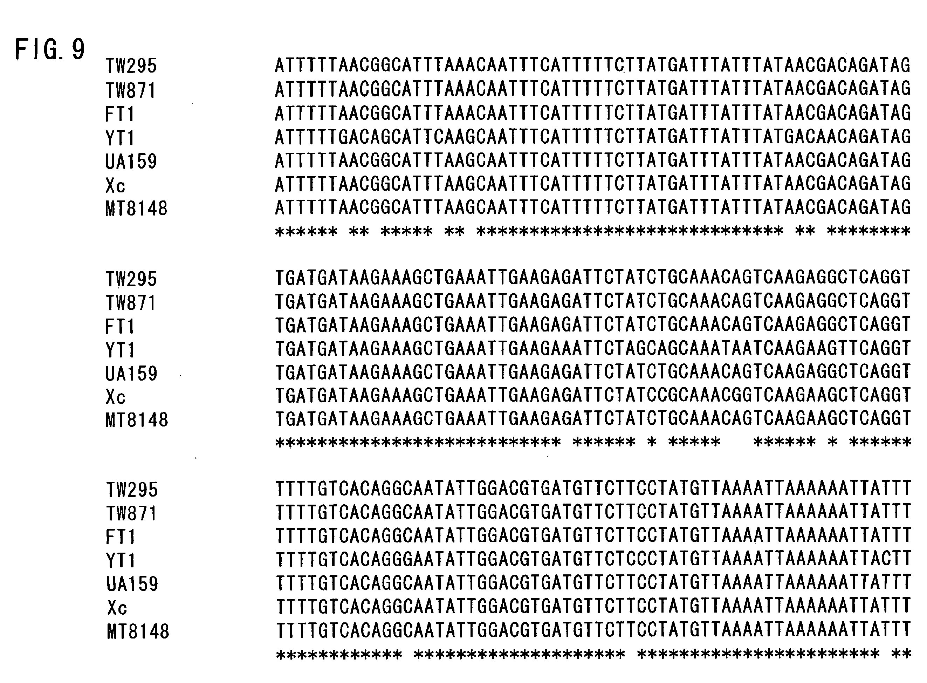

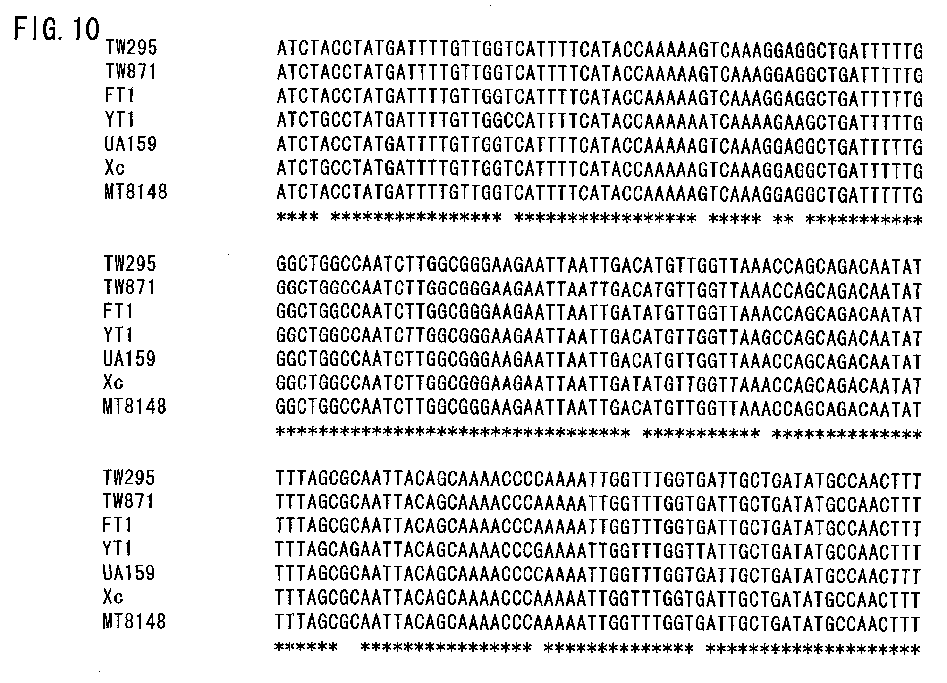

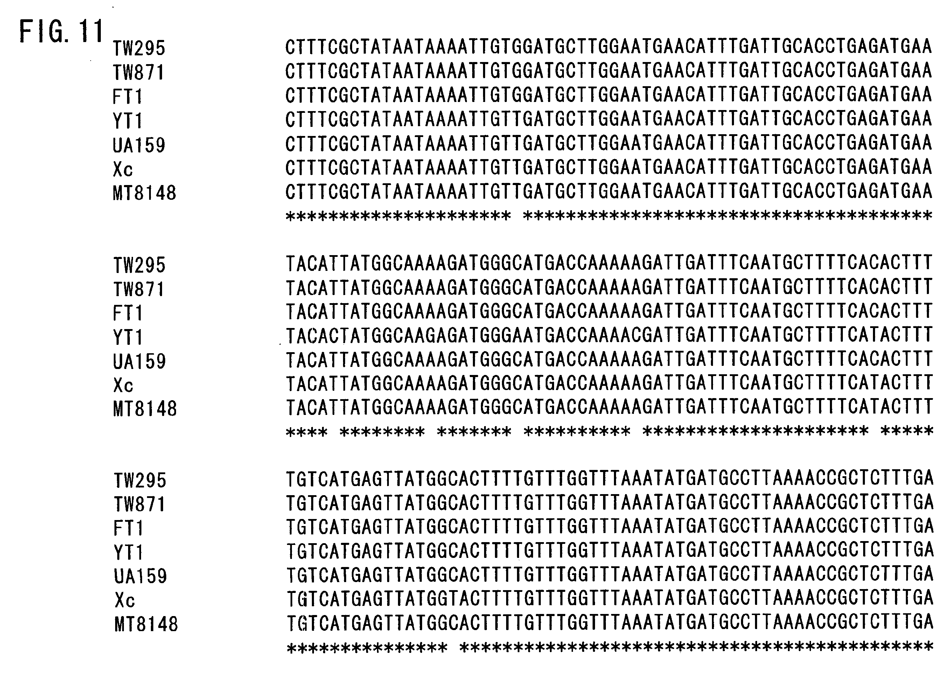

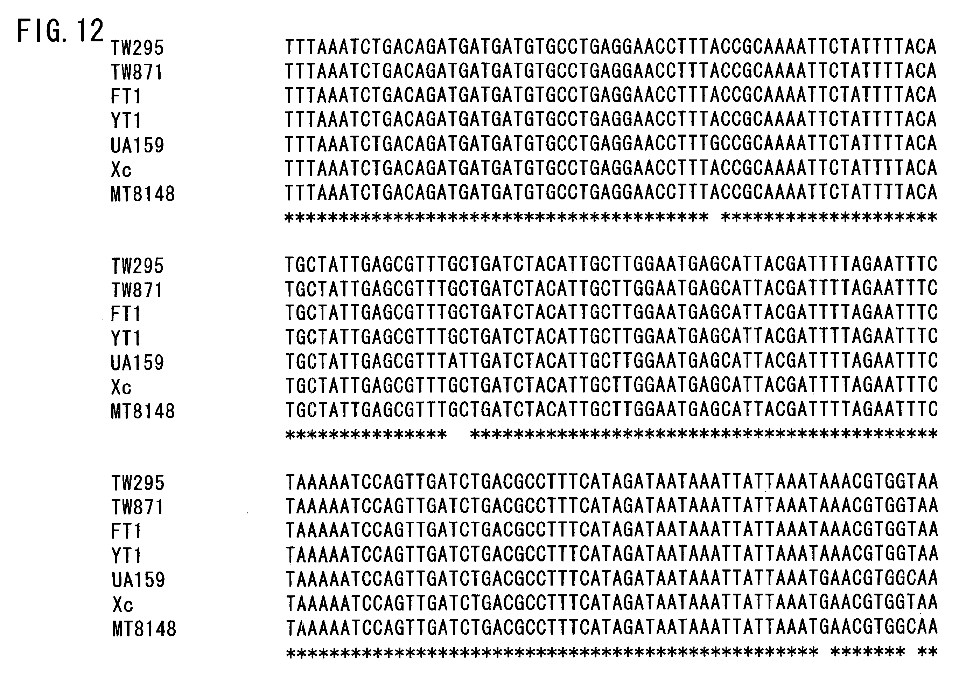

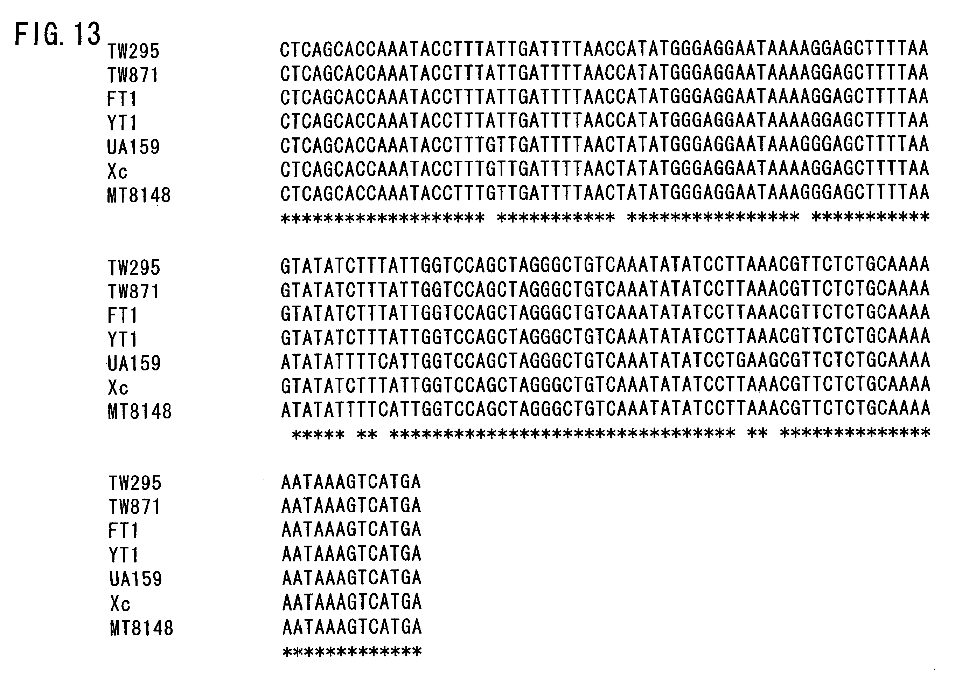

Genomic DNA was extracted from the serotype *k S. mutans* strains (strains TW295, TW871, FT1, YT1). In each strain, gene sequences that encode enzymes associated with the biosynthesis of the serotype-specific polysaccharide antigen were specified, and these sequences were compared with corresponding sequences of MT8148 strain of serotype c. Further, the DNA sequences of the serotype *k S. mutans* strains were compared with the DNA sequences of strains on database (Xc strain: GenBank accession no. AB010970, UA159 strain: GenBank accession no. AE014133), in order to find gene sequences specific to the serotype *k* strains. The serotype *k* strains were found to include many variants in the group of rgp genes, and mutation was commonly present in all *k* type strains at the beginning of *rgpF* gene (Figs. 4 through 13).

### [Example 3: Construction of Simple Identification Method for Serotype k S. mutans Strains]

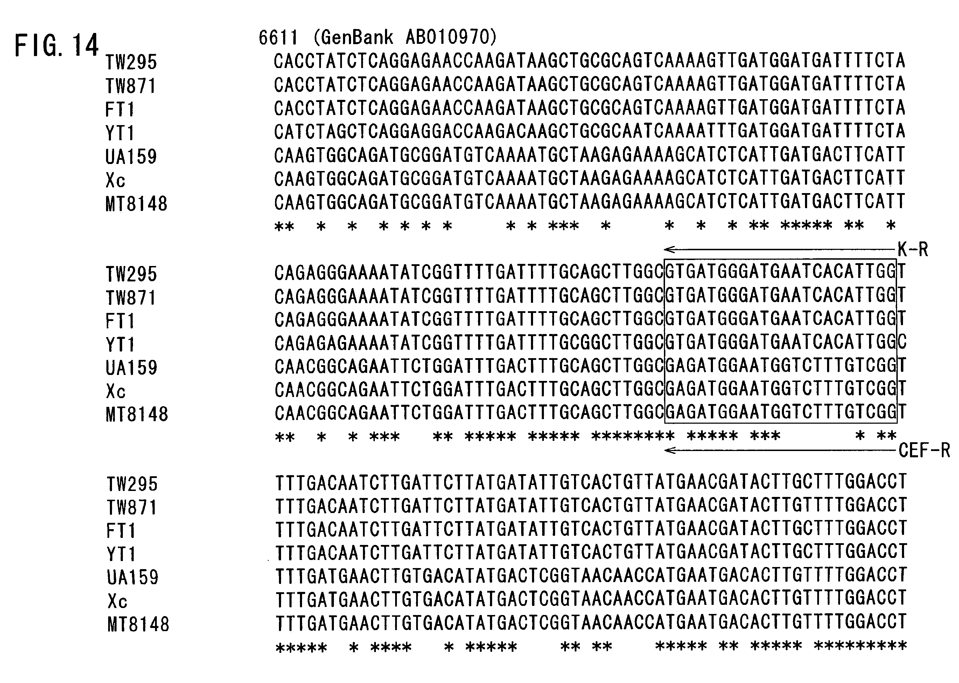

The serotype *k S. mutans* strains had a specific gene sequence in the first one-third of *rgpF* gene as compared with the serotype *c* strains of *S. mutans.* The primers were designed using this region. The forward primer (ATTCCCGCCGTTGGACCATTCC, SEQ ID NO: 8, CRFK-F) was designed based on the sequence that was commonly present for all of the serotype strains (*c-, e-, f-,* and *k*-strains) on the upstream side of the start codon of *rgpF* gene. The reverse primers were designed based on the *k*-specific sequence and *c-, e-, f*-specific sequence (CCAATGTGATTCATCCCATCAC, SEQ ID NO: 9, K-R) and (CCGACAAAGACCATTCCATCTC, SEQ ID NO: 10, DEF-R), respectively (Table 5 and Fig. 14).

**(Table 5. Primers used in the present example and their locations)**

| Primers | Sequence (5' to 3') | Location |
|---|---|---|
| CEFK-F | ATTCCCGCCGTTGGACCATTCC | 6236-6257 |
| K-R | CCAATGTGATTCATCCCATCAC | 6508-6529 |
| CEF-R | CCGACAAAGACCATTCCATCTC | 6508-6529 |

| | | |
|---|---|---|
| The location numbers correspond to the location of the nucleotide sequences obtained from GenBank accession number AB010970 (strain Xc). | | |

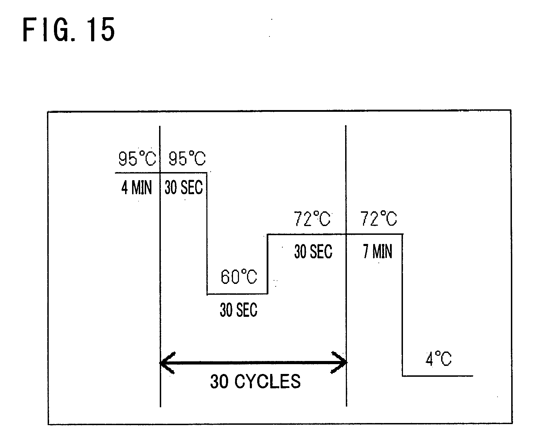

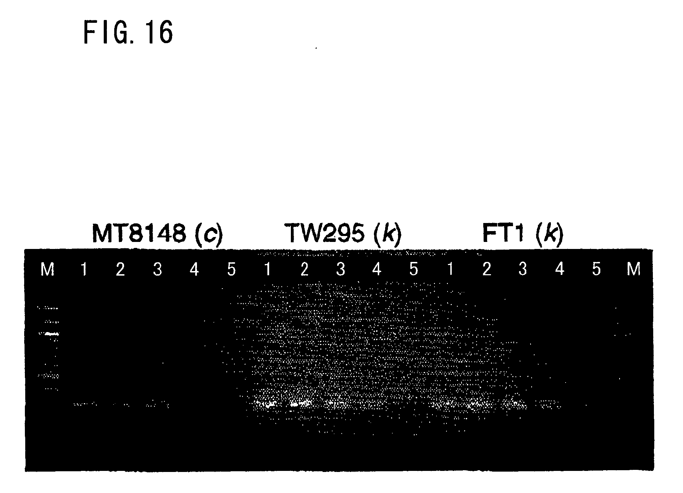

The foregoing primer sets were used to construct a system of PCR amplification in which only the *k* type strains are specifically amplified. More specifically, genomic DNA was extracted from the *k* type *S. mutans* strains (strains TW295, TW871, FT1, and YT1) and saliva samples, and the PCR reaction was performed using the primer set CEFK-F and CEF-R, and the primer set CEFK-F and K-R. The PCR reaction was performed with the following cycling parameters: one cycle consisting of 94°C for 5 minutes; 30 cycles consisting of 94°C for 30 seconds, 60°C for 30 seconds, and 72°C for 30 seconds, and one cycle consisting of 72°C for 7 minutes, as shown in Fig. 15. The PCR products were subjected to electrophoresis in a 1.5% agarose gel. The results are shown in Fig. 16.

### [Example 4: Sensitivity of PCR Assay for Non-Serotype k or Serotype k Strains of S. mutans]

Sensitivity of PCR reaction using the set of primers of CEFK-F and CFF-R and the set of primers of CEFK-F and K-R was studied with titrated cultures of *S. mutans* cells. More specifically, PCR was performed for 10⁸ per ml of strain MT8148(*c*), TW295(*k*), and FT1(*k*) with the set of primers of CEFK-F and K-R. The genomic DNA extracted from the cells was used as the template. The detection limit was determined for simultaneous PCR by the use of known number of bacterial cells (5 x 10⁴, 5 x 10³, 5 x 10², 5 x 10, and 5) diluted in sterile distilled water (Fig. 16).

In Fig. 16, sensitivity of PCR reaction was studied with titrated cultures of 10⁸ per ml of strain MT8148(c), TW295(*k*), and FT1(*k*). The following numbers of cells were added: 5 x 10⁴ (lane 1), 5 x 10³ (lane 2), 5 x 10² (lane 3), 5 x 10 (lane 4), and 5 (lane 5)). M is a molecular size marker (a 100-bp DNA ladder).

The serotype *k*-specific primers produced PCR products in the serotype *k S. mutans* strains (TW295 and FT1), whereas these primers did not produce PCR products in the serotype c strain (MT8148).

The detection limit was found to be highly desirable: 50 to 500 cells with the primer set specific to the serotype *c*/*e*/*f* strains, and 5 to 50 cells with the primer set specific to the serotype *k* strains.

### [Example 5: PCR Assay for the Detection of Serotype k Strains of S. mutans]

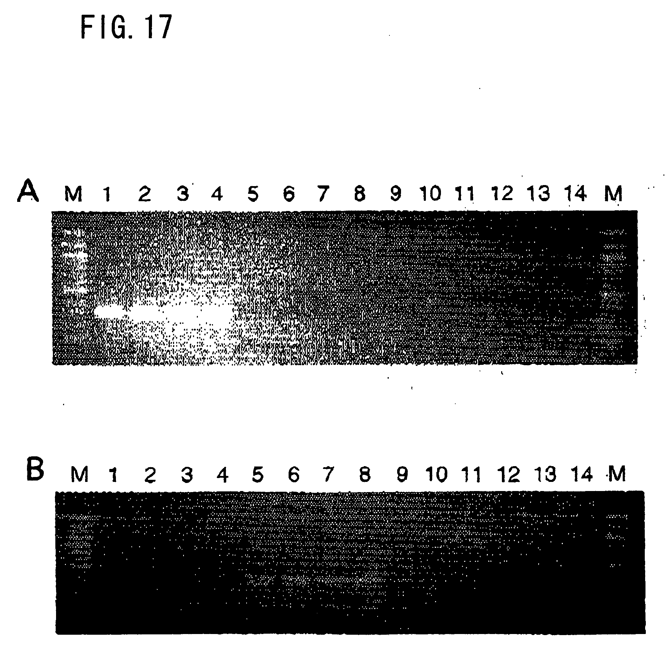

The specificity of the PCR using the set of primers of CEFK-F and CEF-R and the set of primers CEFK-F and K-R was confirmed. As the templates, DNA extracted from the *S. mutans* strains of different serotypes and other Streptococci were used (Figs. 17A and 17B).

In Fig. 17A, the primer set (CEFK-F and CEF-R) specific to serotype *c*/*e*/*f* strains was used to perform the PCR reaction. As the templates, genomic DNA extracted from the following bacteria and saliva samples was used: Genomic DNA extracted from the bacteria of serotype *c*/*e*/*f* strains (lane 1-4); genomic DNA extracted from the bacteria of serotype *k* strains (lanes 5-8); and genomic DNA extracted from saliva samples (lanes 9-14). In Fig. 17B, the primer set (CEFK-F and K-R) (B) specific to serotype *k* strains was used to perform the PCR reaction. As the templates, genomic DNA extracted from the following bacteria and saliva samples was used: Genomic DNA extracted from the bacteria of serotype *c*/*e*/*f* strains (lane 1-4); genomic DNA extracted from the bacteria of serotype *k* strains (lanes 5-8); and genomic DNA extracted from saliva samples (lanes 9-14).

Lane 1, MT8148(c); Lane 2, NN2001(c); Lane 3, NN2002(e); Lane 4, NN2003(*f*); Lane 5, TW295(*k*); Lane 6, TW871(*k*); Lane 7, FT1(*k*); Lane 8, YT1(*k*); Lane 9, *Streptococcus sanguinis* ATCC 10556; Lane 10, *Stresptococcus oralis* ATCC10557; Lane 11, *Stresptococcus gordonii* ATCC 10558; Lane 12, *Streptococcus mitis* ATCC903; Lane 13, *Streptococcus milleri* NCTC10703; Lane 14, *Streptococcus salivarius* HHT.

Though detection of serotype *c*/*e*/*f* strains and serotype *k* strains was specific to the primer set CEFK-F and CEF-R and the primer set CEFK-F and K-R, respectively, neither primer set detected non-*S. mutans* Streptococci. It was therefore found that the primer set CEFK-F and CEF-R and the primer set CEFK-F and K-R could be used to specifically detect serotype *c*/*e*/*f* strains and serotype *k* strains, respectively, and that these primer sets were able to specifically detect only *S. mutans.*

### [Example 6: Identification of Subjects with Serotype k S. mutans Strains by PCR reaction using Saliva Samples]

Expectorated whole saliva (approximately 1 ml) was collected from 200 children or adolescents (2 to 18 years of age [mean age, 7.9 ± 3.6]), who visited the Pedodontics Clinic in Osaka University Dental Hospital in January and February 2004. The saliva samples were processed by PCR assay by the method reported by Hoshino et al., (2004) with some modifications. Briefly, non-stimulated whole saliva was collected in a sterile tube and kept on ice. Bacterial cells were collected in a micro-centrifuge tube from 500 µl of saliva at 16,000 x g for 5 min, and treated in a microwave oven at 500 W for 5 min, which were digested in N-Acetylmuramidase SG (Seikagaku Corp., Tokyo, Japan) at 50°C for 1 h. Then, 80 µl of nucleilysis solution (Promega) was added and incubated at 80°C for 5 min, followed by the addition of 60 µl of protein precipitation solution (Promega). The proteins were removed by centrifugation at 16,000 x g for 3 min, and the DNA was purified by phenol-chloroform extraction and ethanol precipitation. The extracted DNA was dissolved in 50 µl of TE buffer (10 mM Tris-HCl, 1 mM EDTA; pH 8.0). This method of DNA extraction can also be used to extract bacterial genome from plaque.

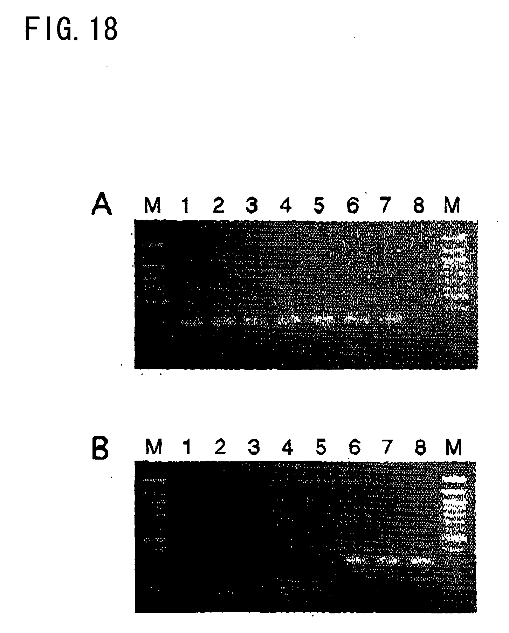

By the PCR that used the extracted DNA as the templates, serotype *c*/*e*/*f* strains (A) and serotype *k* strains (B) were detected with the set of primers CEFK-F and CEF-R and the set of primers CEFK-F and K-R, respectively (Figs. 18A and 18B). Among 200 clinical samples, the 190 samples showed the positive reaction for serotype non-k (*c*/*e*/*f*) specific set of primers (A), and the negative reaction for serotype *k* specific set of primers (B). On the other hand, ten samples showed the positive reaction for both of the serotype *k*-specific (B) and non-specific set of primers (A).

While the test showed positive for the serotype non-*k* (c/e/f) specific set of primers CEFK-F and CEF-R (A), Fig. 18A shows 5 representative subjects among 190 samples with negative for serotype *k* specific set of primers (B). Referring to Fig. 18B, the set of primers CEFK-F and K-R was used for the detection of serotype *k* strains. Lane 1 through Lane 5; 5 representative subjects among 190 samples with negative for serotype *k* specific set of primers. Lane 6 through Lane 8; subjects with positive reaction for serotype *k* specific set of primers.

As described above, *k*-specific primers yielded PCR products in serotype *k S. mutans* strains (strains TW295, TW871, FT1, SU1, YK1, YT1, and AT1) and clinical isolates of *S. mutans* (NN2000 series, 78 c-strains, 17 *e*-strains, and 3 *f*-strains), whereas *c-, e-,* or *f*-specific primers produced no PCR products in these strains. The *c*-, *e*-, or *f*-specific primers produced PCR products in 98 strains of serotype *c-, e-,* or *f-S. mutans*, whereas *k*-specific primers produced no PCR products in these strains.

### [Example 7]

*S. mutans* are classified into *c*, *e*, and *f* serotypes based on the chemical composition of their cell surface polysaccharide (Linzer et al., 1987). The serotype specific polysaccharide of *S. mutans* is known to consist of rhamnose-glucose polymers, with a backbone of rhamnose and side chains of α- or β-linked glucosidic residues. In a previous study of the inventors, 2 serologically untypable strains from the blood of patients with bacteremia (strain TW295) and IE (strain TW871) were shown to lack a glucose side chain in the serotype specific polysaccharide (Fujiwara et al., 2001).

As described in Example 1, a serotype *k* strain of MT8148GD exhibited high levels of sucrose-dependent adhesion, however, the adhesion was significantly lower than that of the parent strain MT8148 (serotype *c*).

Recently, a serotype specific polysaccharide was demonstrated to play important roles in streptococcal adherence to human monocytic and fibroblastic cells, and was speculated to be the most efficient cytokine-stimulating component (Engels-Deutsch et al., 2003). However, the relationship between serotype specific polysaccharide and dental caries remains to be elucidated.

The purpose of the present example was to analyze the role of the glucose-side chain of serotype specific polysaccharide in the cariogenicity of the newly elucidated serotype *k* strains of *S. mutans.*

### (A) S. mutans Strains

*S. mutans* strains used in the present example are listed in Table 6. An oral isolate strain of MT8148(c) was selected from the stock culture collection in the library of the inventors. Strains FT1(*k*), SU1(*k*), and YK1(*k*) were also isolated from the oral cavity of Japanese children. In addition, strains YT1 was isolated from the oral cavities of a 6-year old healthy boy, and confirmed to be *S. mutans* serotype *k* according to the methods described in Example 1. Strains MT8148R and YK1R were made resistant to streptomycin by repeated passages in increasing concentrations of the antibiotic, up to a final concentration of 1500 µg of streptomycin per mL of agar medium (Ooshima et al., 2000). Strain MT8148GD, a *glu*A-inactivated isogenic mutant strain of MT8148, was constructed as in Example 1.

**(Table 6. Bacterial strains used in the present example)**

| Strain | Serotype | Features |
|---|---|---|
| MT8148 | c | Oral isolate from healthy child |
| MT8148GD | *k* | Em^{r}; strain MT8148 carrying *emr* inserted into *gluA* |
| MT8148R | *c* | Sm^{r}; repeated passages of MT8148 in Increasing concentrations of streptomycin |
| MT8148RGD | *c* | Sm^{r}, Em^{r}; strain MT8148R carrying *emr* inserted into *gluA* |
| TW295 | *k* | Blood isolate from 59-year-old male with bacteremia following a tooth extraction procedure |
| TW871 | *k* | Blood isolate from 45-year-old female with infective endocarditis complicated with subarachnoid hemorrhage |
| FT1 (NN2011) | *k* | Oral isolate from 3-year-old healthy girl |
| SU1 (NN2029) | *k* | Oral isolate from 10-year-old healthy girl |
| YK 1 | *k* | Oral isolate from 6-year-old girl with Down's syndrome |
| YK1R | *k* | Sm^{r}; repeated passages of YK1 in Increasing concentrations of streptomycin |
| YT1 | *k* | Oral isolate from 6-year-old healthy boy |

| | | |
|---|---|---|
| Em^{r}: erythromycin resistant, Sm^{r}: streptomycin resistant | | |

### (B) In vitro Analysis of Cariogenic Properties

*S. mutans* sucrose-dependent adhesion to glass tubes and sucrose-independent adhesion to saliva-coated hydroxyapatite (SHA) were analyzed as described previously (Ooshima et al. 2001; Nakano et al. 2002). Cellular hydrophobicity and dextran-binding activity were also measured using a method described previously (Fujiwara et al., 2001; Nakano et al., 2002).

Intergroup differences of various factors were estimated by a statistical analysis of variance (ANOVA) for factorial models (Table 7).

Table 7 shows the biological properties from the *in vitro* analysis cariogenicity. MT8148GD showed a significantly lower rate of sucrose-dependent adhesion to a glass surface, sucrose-independent adhesion to SHA, dextran-binding activity, CA-GTF activity, and CF-GTF activity (P < 0.001), whereas there were no significant differences in these properties between FT1 and FT1GD.

**Table 7. Biological properties of serotype k strains**

| Strains | Sucuse- dependent adhesion [Mean±SD(%)] | Sucrose- independent adhesion [Mean±SD(%) | Dextran -binding activity [Mean±SD] | CA-GIF activity [Mean±SD (mU/mL)] | CF-GTF activity [Mean±SD (mU/mL)] |
|---|---|---|---|---|---|
| MT8148(*c*) | 91.5±0.5 | 100.0±2.1 | 0.15±0.03 | 73.0±3.9 | 95.0±2.8 |
| MT8148GD(*k*) | 70.5±3.8^{a} | 88.6±1.7^{a} | 0.02±0.01^{a} | 29.4±0.8^{a} | 59.8±0.9^{a} |
| TW295(*k*) | 70.4±3.1^{a} | 77.9±2.0^{a} | 0.03±0.01^{a} | 34.9±1.6^{a} | 25.1±0.3^{a} |
| TW871(*k*) | 75.1±0.9^{a} | 56.0±3.8^{a} | 0.09±0.04^{a} | 61.2±1.5^{a} | 56.0±2.3^{a} |
| FT1(*k*) | 83.1±2.1^{a} | 89.6±0.3^{a} | 0.05±0.00^{a} | 38.6±0.3^{a} | 35.7±0.7^{a} |
| FIFIGD(*k*) | 81.8±0.4^{a} | 89.8±0.2^{a} | 0.05±0.01^{a} | 41.2±1.2^{a} | 38.0±1.1^{a} |
| SU1(*k*) | 85.4±0.9^{a} | 105.9±4.1 | 0.07±0.02^{a} | 48.3±3.7^{a} | 33.0±0.2^{a} |
| YK1(*k*) | 82.7±1.2^{a} | 95.0±3.4 | 0.03±0.00^{a} | 47.4±2.5^{a} | 38.4±0.6^{a} |
| YT1(*k*) | 80.8±1.4^{a} | 87.7±9.7^{a} | 0.02±0.01^{a} | 28.9±1.7^{a} | 30.4±0.4^{a} |

| | | | | | |
|---|---|---|---|---|---|
| There were significant differences between MT8148 and the other strains by Fisher's PLSD analysis (^{a}*P*<0.001). | | | | | |

### (C) Expression of GTFs

Immunoblotting of GTFs was performed to assess the amount of expressed protein using cell-associated (CA)-and cell-free (CF)-GTF antisera, which were generated in a previous study of the inventors. The test organisms were grown in Brian Heart Infusion (BHI) broth (Difco Laboratories, Detroit, MI, USA) at 37°C to an optimum density of 1.0 at 550 nm. The bacterial cells and supernatant, concentrated by ammonium sulfate precipitation, were dissolved in SDS gel loading buffer. An equal amount of each protein was separated by 7% SDS polyacrylamide gel electrophoresis and then transferred onto a polyvinylidene difluoride membrane (Immobilon; Millipore, Bedford, MA, USA). The transferred protein bands were reacted with rabbit antibodies against CA- or CF-GTF, and then visualized using an alkaline phosphatase-conjugated anti-rabbit immunoglugulin G antibody (New England Biolabs, Beverly, MA, USA) and 5-bromo-4-chloro-3-indolylphosphate-nitroblue tetrazolium substrate (Moss Inc., Pasadena, MD, USA). In addition, the ratio of the intensity of the expression of GTFB to that of GTFC in each strain was estimated by relative densitometric analysis of the reaction bands using NIH image software (National Technical Information Service, Springfield, VA, USA).

GTFB, GTFC, and GTFD were found expressed in all of the tested strains, however, the intensity of GTFB was significantly stronger in MT8148 than in any of the other strains. The ratio of GTFB expression intensity to that of GTFC of MT8148 was 1.80±0.15, which was significantly higher than that of MT8148GD (0.80±0.11) and those of the other serotype *k* blood or oral isolates (ratios ranging from 0.17 - 0.81) (*P* < 0.001). In contrast, there were no significant differences in GTFD expression intensity among all the tested strains.

### (D) Measurement of CA- and CF-GTF Activities

CA- and CF-GTF activities were estimated by [¹⁴C] glucan synthesis from [¹⁴C] sucrose, using a method described previously (Matsumoto et al., 2003). One unit of GTF activity was defined as the amount of enzyme needed to incorporate 1 mmol of glucose residue from sucrose into glucan in 1 minute.

The serotype *k* blood isolates TW295 and TW871, and the 3 oral isolates SU1, YK1, and YT1, each showed a significantly lower sucrose-dependent adhesion rate, dextran binding activity, CA-GTF activity, and CF-GTF activity than MT8148 (*P*<0.001). As for sucrose-independent adhesion to SHA, the rates of the tested serotype *k* strains except for strains SU1 and YK1 were significantly lower than that of MT8148 (P<0.001). In addition, there were no significant differences in cellular hydrophobicity between MT8148 and the other tested strains.

### (E) Caries Inducing Experiments in Rats

Caries-inducing activities were examined in 36 specific pathogen-free (SPF) Sprague-Dawley rats (12 rats per group) (Charles River Inc., Osaka, Japan) according to a method described previously (Nakano et al., 2002). The 3 tested strains (MT8148R, MT8148RGD, and YK1R) at 5 x 10⁹ CFU were individually orally infected into rats aged 18 to 22 days, after which plaque and caries scores for each rat were evaluated at the age of 72 days.

Intergroup differences factors were estimated by a statistical analysis of variance (ANOVA) for factorial models.

In the rats, serotype *k* induced evident dental caries. However, there were no significant differences in caries scores or plaque index between strain MT8148R, its mutant MT8148RGD, and oral isolate YK1R (Table 8).

**Table 8. Caries inducing activity of serotype k strains in rats**

| Strains | Number of rats | Plaque Index [Mean±SE] | Caries Scores Smooth | [Mean ± SE] Total |
|---|---|---|---|---|
| MT8148R (*c*) | 12 | 0.9±0.1 | 13.0±0.8 | 55.0±2.8 |
| MT8148RGD (*k*) | 12 | 0.9±0.1 | 13.4±1.6 | 53.6±5.3 |
| YK1R(*k*) | 12 | 0.8±0.1 | 14.5±1.5 | 54.1±4.6 |

### (F) Discussion

In the present example, the biological properties of MT8148GD, constructed by insertional inactivation of the *gluA* gene, which encodes the enzyme that catalyzes the production of UDP-D-glucose, the immediate precursor of the glucose side chain donor, were compared with those of the parent strain MT8148. Since UDP-D-glucose is reported to be important for the viability of *S. mutans* in a low pH environmental condition, we speculated that inactivation of the *gluA* gene itself may cause an alteration of the properties relating to cariogenicity. Therefore, we constructed an additional mutant strain of serotype *k* strain FT1 (FT1GD), in which the *gluA* gene was insertionally inactivated. Results showed that there were no significant differences in the biological properties between FT1 and FT1GD (Table 7), indicating that inactivation of the *gluA* gene itself is not associated with the properties measured in the *in vitro* assay. Therefore, it was concluded that the presence of the glucose side chain of the serotype specific polysaccharide would be important for higher sucrose-dependent adhesion, sucrose-independent adhesion, dextran-binding activity, and CA- and CF-GTF activities.

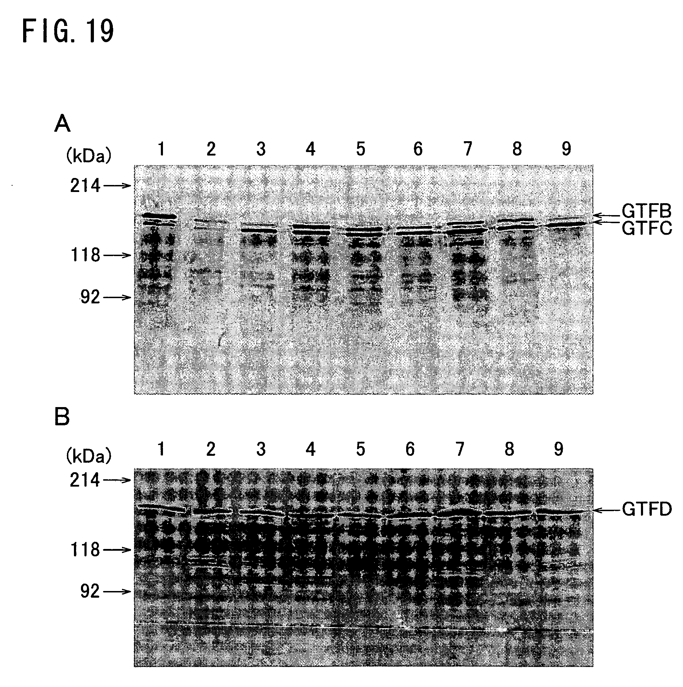

A reduction of CA- and CF-GTF activity was prominent in the serotype *k* clinical isolates, as well as in MT8148GD (Table 7). Further, the CA-and CF-GTF activities in the serotype *k* clinical isolates were significantly lower than those in 30 clinical isolates of *S. mutans* (16 serotype c strains, 8 serotype e strains, and 6 serotype *f* strains). The optimal GTFB/GTFC/GTFD ratio necessary for appropriate colonization *in vitro* was determined in a previous study of the inventors, and deviation from this ratio could compromise the adherence properties and structure of plaque biofilm (Idone et al., 2003). Western blot analysis in the present study indicated that the expression of GTFB tended to be lower in the serotype *k* strains than in MT8148 (Figs. 19A and 19B).

Referring to Fig. 19A, whole cells (A) were separated by 7% SDS polyacrylamide gel electrophoresis and the transferred protein bands on a PVDF membrane were reacted with rabbit antibodies against CA-GTF of *S. mutans.* In Fig. 19B, the culture supernatant concentrated by ammonium sulfate precipitation were separated by 7% SDS polyacrylamide gel electrophoresis and the transferred protein bands on a PVDF membrane were reacted with rabbit antibodies against CF-GTF of *S. mutans.* Lane 1, strain MT8148; lane 2, strain MT8148GD; lane 3, strain TW295; lane 4, strain TW871; lane 5, strain FT1; lane 6, strain FT1GD; lane 7, strain SU1; lane 8, strain YK1; lane 9, strain YT1.

GTFB is thought to be responsible for the majority of water-insoluble glucan synthesis, which is an important virulence factor in initial caries development, as it caused an increased level of adherence and accumulation of mutans streptococci in the plaque of young children (Matto-Graner et al., 2000). Thus, the lower CA-GTF activity of the serotype *k* strains may be attributable to the lower expression of GTFB, while its localization on the cell surface might be correlated with the presence of a glucose side chain of serotype specific polysaccharide. Taken together, these results suggest that a defect of glucose side chain in the serotype specific polysaccharide of *S. mutans* may be associated with cariogenicity of the organism, though to a lesser extent than the other major surface structures of *S. mutans,* such as GTFs, PAc, and Gbps.

The present invention is not limited to the description of the embodiments above, but may be altered by a skilled person within the scope of the claims. An embodiment based on a proper combination of technical means disclosed in different embodiments is encompassed in the technical scope of the present invention. The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

The specific embodiments and examples of implementation discussed in the foregoing BEST MODE FOR CARRYING OUT THE INVENTION section serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRICAL APPLICABILITY

*S. mutans* strains involved in the onset of infective endocarditis are not easily distinguishable from other strains of *S. mutans.* The present invention provides ways to conveniently determine *S. mutans* strains, designated as new serotype *k* strains, that are likely to be involved in the onset of infective endocarditis, and therefore the invention is applicable to a wide range of fields, including dentistry and other medical applications, as well as pharmaceutical and test industries.

The invention provides a method for effectively and efficiently determining whether a subject carries serologically untypable strains of *S. mutans.* This is important in the prevention of potentially fatal IE.

## Claims

1. A polynucleotide that encodes a polypeptide that reduces the amount of glucose side chain of a polysaccharide antigen specific to *Streptococcus mutans,*
wherein the polynucleotide comprises:
a base sequence of any of SEQ ID NO: 1 through 4; or
a base sequence with the deletion, substitution, or addition of one or more bases in the base sequence of any of SEQ ID NO: 1 through 4.

2. A polynucleotide that encodes a polypeptide that reduces the amount of glucose side chain of a polysaccharide antigen specific to *Streptococcus mutans,*
wherein the polynucleotide comprises:
a polynucleotide of a base sequence of any of SEQ ID NO: 1 through 4; or
a polynucleotide that hybridizes under stringent conditions with a polynucleotide having a complementary base sequence to the polynucleotide of the base sequence of any of SEQ ID NO: 1 through 4.

3. An oligonucleotide that comprises a base sequence, or a complementary sequence thereof, with at least 12 contiguous bases of a base sequence of any of SEQ ID NO: 1 through 4.

4. An oligonucleotide of claim 3, which comprises a base sequence of any of SEQ ID NO: 8 through 10.

5. A polypeptide that is encoded by a polynucleotide of claim 1 or 2.

6. A *Streptococcus mutans* strain with a reduced amount of glucose side chain in a polysaccharide antigen specific to *Streptococcus mutans.*

7. A *Streptococcus mutans* strain of claim 6, which comprises a polynucleotide of claim 1 or 2.

8. A *Streptococcus mutans* strain of claim 6, which expresses a polypeptide of claim 5.

9. An antibody that specifically binds to a *Streptococcus mutans* strain of *any of* claims 6 through 8.

10. A method for detecting a *Streptococcus mutans* strain in a subject sample, comprising the steps of:
separating bacteria from the subject sample;
extracting genomic DNA or total RNA of the bacteria separated from the subject sample; and
carrying out a PCR reaction, using the genomic DNA or total RNA as a template, and using an oligonucleotide of claim 3 or 4 as a primer.

11. A method for detecting a *Streptococcus mutans* strain in a subject sample set forth in claim 10, wherein the tissue sample is obtained from blood, saliva, or plaque.

12. A method for detecting a *Streptococcus mutans* strain in a subject sample set forth in claim 10 or 11,
wherein the primers are an oligonucleotide of a base sequence of SEQ ID NO: 8, and an oligonucleotide of a base sequence of SEQ ID NO: 9 or 10.

13. A method for detecting a *Streptococcus mutans* strain in a subject sample, comprising the steps of:
separating bacteria from the subject sample;
extracting genomic DNA or total RNA of the bacteria separated from the subject sample; and
carrying out a hybridization reaction for the genomic DNA or total RNA, using an oligonucleotide of claim 3 or 4 as a probe.

14. A method for detecting a *Streptococcus mutans* strain in a subject sample set forth in claim 13, wherein the tissue sample is obtained from blood, saliva, or plaque.

15. A method for detecting a *Streptococcus mutans* strain in a subject sample set forth in claim 13 or 14, wherein the oligonucleotide has a base sequence of any of SEQ ID NO: 8 through 10.

16. A method for detecting a *Streptococcus mutans* strain in a subject sample set forth in *any of* claims 10 through 15, wherein the step of separating bacteria uses an antibody of claim 9.

17. A method for detecting a *Streptococcus mutans* strain in a subject sample, comprising the steps of:
separating bacteria from the subject sample;
incubating the separated bacteria with an antibody of claim 9; and
detecting bacteria that have bound to the antibody.

18. A method for determining a serotype of a *Streptococcus mutans* strain in a subject sample, the method comprising using a method of *any of* claims 10 through 17.

19. A screening method of a *Streptococcus mutans* strain, the method comprising using a method of any one claims 10 through 17.

20. A *Streptococcus mutans* strain, which is obtained by a screening method of claim 19.

21. A kit for detecting a *Streptococcus mutans* strain, the kit comprising an oligonucleotide of claim 3 or 4.

22. A kit for detecting a *Streptococcus mutans* strain set forth in claim 21, wherein the oligonucleotide comprises a base sequence of SEQ ID NO: 9.

23. A kit for detecting a *Streptococcus mutans* strain set forth in claim 22, the kit further comprising an oligonucleotide of a base sequence of SEQ ID NO: 8.

24. A kit for detecting a *Streptococcus mutans* strain set forth in claim 23, the kit further comprising an oligonucleotide of a base sequence of SEQ ID NO: 10.

25. A kit for detecting a *Streptococcus mutans* strain set forth in *any of* claims 21 through 24, which is used for a PCR reaction or a hybridization reaction.

26. A kit for detecting a *Streptococcus mutans* strain, the kit comprising an antibody of claim 9.

27. A method for producing an antibody of claim 9, comprising the step of injecting a *Streptococcus mutans* strain of *any of* claims 6 through 8, suspended in a buffer, intravenously into the auricular vein of rabbits repeatedly for 5 consecutive days.

28. A method for producing an antibody set forth in claim 27, further comprising the step of repeating immunization of the *Streptococcus mutans* strain, suspended in a phosphate-buffered saline, one week after the injecting step and for another 2 weeks, 5 times each week.

29. A bacteria detecting tool, which comprises an oligonucleotide, fixed on a substrate, that include a base sequence with at least 12 contiguous bases of a base sequence of any of SEQ ID NO: 1 through 4.

30. A bacteria detecting tool of claim 29, wherein the oligonucleotide comprises an oligonucleotide of claim 3 or 4.


SEQUENCE LISTING

<110> SUNTORY LIMITED

<120> NOVEL SEROTYPE STREPTOCOCCUS MUTANS AND UTILIZATION OF THE SAME

<130> SU0401

<140> PCT/JP2004/019631
<141> 2004-12-28

<150> US 60/533,076
<151> 2003-12-30

<150> JP 2004-106825
<151> 2004-03-31

<160> 10

<170> PatentIn Ver. 2.1

<210> 1
<211> 1752
<212> DNA
<213> Streptococcus mutans

<400> 1
atgaaaagac tacttttgta tgtgcatttt aataaatata atcgtgtgag ttctcatgtt 60
tactaccaac taacacaaat gcgcccctta ttttcaagag tagttttcat cacaaatagt 120
cacctatctc aggagaacca agataagctg cgcagtcaaa agttgatgga tgatttctta 180
cagagggaaa atatcggttt tgattttgca gcttggcgtg atgggatgaa tcacattggt 240
tttgacaatc ttgattctta tgatattgtc actgttatga acgatacttg ctttggacct 300
ttgtgggatg ttaaggagca ttacctgtcc tatgaaaagc aggacgagat tgatttttgg 360
ggattgacta ataatcgtgc gactaagcag tttaaggaac atattcaaag ctactttatt 420
acctttaaaa aagctgttat tcaatcggag gcctttcata atttttggga gaacatccaa 480
aatcatgcag atattcaacg tgtcattgat gattacgaaa ctcaggtgac gacaacgctt 540
ttggatgctg gttttcaata tgatgtcgtt tttgatacga ccaaagaaga tgcttcgcgt 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ctaatcgttt cgcacatgtc agaaatcaat 780
tatcctgatt ttagttattt attgggtcac aaatatgtca agaaaaggga aagagttgat 840
ttaaagaatc aaaaagctgc ggttcatctc catgtgtttt atgtggattt gctggaagaa 900
tttttaacgg catttaaaca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa ccccaaaatt ggtttggtga ttgctgatat gccaactttc 1260
tttcgctata ataaaattgt ggatgcttgg aatgaacatt tgattgcacc tgagatgaat 1320
acattatggc aaaagatggg catgaccaaa aagattgatt tcaatgcttt tcacactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 2
<211> 1752
<212> DNA
<213> Streptococcus mutans

<400> 2
   atgaaaagac tacttttgta tgtgcatttt aataaatata atcgtgtgag ttctcatgtt 60
   tactaccaac taacacaaat gcgcccctta ttttcaagag tagttttcat cacaaatagt 120
   cacctatctc aggagaacca agataagctg cgcagtcaaa agttgatgga tgatttctta 180
   cagagggaaa atatcggttt tgattttgca gcttggcgtg atgggatgaa tcacattggt 240
   tttgacaatc ttgattctta tgatattgtc actgttatga acgatacttg ttttggacct 300
   ttgtgggatg ttaaggagca ttacctgtcc tatgaaaagc aggacgagat tgatttttgg 360
   ggattgacta ataatcgtgc gactaagcag tttaaggaac atattcaaag ctactttatt 420
   acctttaaaa aagctgttat tcaatcggag gcctttcata atttttggga gaacatccaa 480
   aatcatgcag atattcaacg tgtcattgat gattacgaaa ctcaggtgac gacaacgctt 540
   ttggatgctg gttttcaata tgatgtcgtt tttgatacga ccaaagaaga tgcttcgcat 600
   atgcttcatg ccgacttctc ttactataat ccaacagcta ttttgaatca tagggtgccc 660
   tttatcaagg ttaaagcgat tgacaataat caacatatta cgccctatct tttaaatgat 720
   attcaaaaga attcgaccta tcctattgat ttaatcgttt cgcacatgtc agaaatcaat 780
   tatcctgatt ttagttattt attgggtcac aaatatgtca agaaaaggga aagagttgat 840
   ttaaagaatc aaaaagctgc ggttcatctc catgtgtttt atgtggattt gctggaagaa 900
   tttttaacgg catttaaaca atttcatttt tcttatgatt tatttataac gacagatagt 960
   gatgataaga aagctgaaat tgaagagatt ctatctgcaa acagtcaaga ggctcaggtt 1020
   tttgtcacag gcaatattgg acgtgatgtt cttcctatgt taaaattaaa aaattattta 1080
   tctacctatg attttgttgg tcattttcat accaaaaagt caaaggaggc tgatttttgg 1140
   gctggccaat cttggcggga agaattaatt gacatgttgg ttaaaccagc agacaatatt 1200
   ttagcgcaat tacagcaaaa cccaaaaatt ggtttggtga ttgctgatat gccaactttc 1260
   tttcgctata ataaaattgt ggatgcttgg aatgaacatt tgattgcacc tgagatgaat 1320
   acattatggc aaaagatggg catgaccaaa aagattgatt tcaatgcttt tcacactttt 1380
   gtcatgagtt atggcacttt tgtttggttt aaatatgatg ccttaaaacc gctctttgat 1440
   ttaaatctga cagatgatga tgtgcctgag gaacctttac cgcaaaattc tattttacat 1500
   gctattgagc gtttgctgat ctacattgct tggaatgagc attacgattt tagaatttct 1560
   aaaaatccag ttgatctgac gcctttcata gataataaat tattaaataa acgtggtaac 1620
   tcagcaccaa atacctttat tgattttaac catatgggag gaataaaagg agcttttaag 1680
   tatatcttta ttggtccagc tagggctgtc aaatatatcc ttaaacgttc tctgcaaaaa 1740
   ataaagtcat ga 1752

<210> 3
<211> 1752
<212> DNA
<213> Streptococcus mutans

<400> 3
   atgaaaagac tacttttgta tgtgcatttt aataaatata atcgtgtgag ttctcatgtt 60
   tactaccaac taacacaaat gcgcccctta ttttcaagag tagttttcat cacaaatagt 120
   cacctatctc aggagaacca agataagctg cgcagtcaaa agttgatgga tgattttcta 180
   cagagggaaa atatcggttt tgattttgca gcttggcgtg atgggatgaa tcacattggt 240
   tttgacaatc ttgattctta tgatattgtc actgttatga acgatacttg ctttggacct 300
   ttgtgggatg ttaaggagca ttacctgtcc tatgaaaagc aggacgagat tgatttttgg 360
   ggattgacta ataatcgtgc gactaagcag tttaaggaac atattcaaag ctactttatt 420
   acctttaaaa aagctgttat tcaatcggag gcctttcata atttttggga gaacatccaa 480
   aatcatgcag atattcaacg tgtcattgat gattacgaaa ctcaggtgac gacaacgctt 540
   ttggatgctg gttttcaata tgatgtcgtt tttgatacga ccaaggaaga tgcttcgcat 600
   atgcttcatg cagacttctc ttactataat ccaacagcta ttttgaatca tagggtgccc 660
   tttatcaagg ttaaagcgat tgacaataat caacatatta cgccctatct ttcaaatgat 720
   attcaaaaga attcgaccta tcctattgat ttaatcgttt cgcacatgtc agaaatcaat 780
   tatcctgatt ttagttattt attgggtcac aaatatgtca agaaaaggga aagagttgat 840
   ttaaagaatc aaaaagctgc ggttcatctc catgtgtttt atgtggattt gctggaagaa 900
   tttttaacgg catttaaaca atttcatttt tcttatgatt tatttataac gacagatagt 960
   gatgataaga aagctgaaat tgaagagatt ctatctgcaa acagtcaaga ggctcaggtt 1020
   tttgtcacag gcaatattgg acgtgatgtt cttcctatgt taaaattaaa aaattattta 1080
   tctacctatg attttgttgg tcattttcat accaaaaagt caaaggaggc tgatttttgg 1140
   gctggccaat cttggcggga agaattaatt gatatgttgg ttaaaccagc agacaatatt 1200
   ttagcgcaat tacagcaaaa cccaaaaatt ggtttggtga ttgctgatat gccaactttc 1260
   tttcgctata ataaaattgt ggatgcttgg aatgaacatt tgattgcacc tgagatgaat 1320
   acattatggc aaaagatggg catgaccaaa aagattgatt tcaatgcttt tcacactttt 1380
   gtcatgagtt atggcacttt tgtttggttt aaatatgatg ccttaaaacc gctctttgat 1440
   ttaaatctga cagatgatga tgtgcctgag gaacctttac cgcaaaattc tattttacat 1500
   gctattgagc gtttgctgat ctacattgct tggaatgagc attacgattt tagaatttct 1560
   aaaaatccag ttgatctgac gcctttcata gataataaat tattaaataa acgtggtaac 1620
   tcagcaccaa atacctttat tgattttaac catatgggag gaataaaagg agcttttaag 1680
   tatatcttta ttggtccagc tagggctgtc aaatatatcc ttaaacgttc tctgcaaaaa 1740
   ataaagtcat ga 1752

<210> 4

<211> 1752
<212> DNA
<213> Streptococcus mutans

<400> 4
atgaaaagac tgcttttgta tgtgcatttt aataaatata atcgtgtgag ttcccatgtt 60
tactaccaac tgacacaaat gcgcccctta ttttcaagag tagttttcat cacaaatagc 120
catctagctc aggaggacca agacaagctg cgcaatcaaa atttgatgga tgattttcta 180
cagagagaaa atatcggttt tgattttgcg gcttggcgtg atgggatgaa tcacattggc 240
tttgacaatc ttgattctta tgatattgtc actgttatga acgatacttg ctttggacct 300
ttgtgggatg ttaaggatta ttacctgtcc tatgaaaagc aagatgaagt tgatttttgg 360
ggattgacca ataatcgtgc gactaagcag tttaaggagc atattcaaag ctactttatt 420
acttttaaaa aggctgttat tcaatcaaat gcctttcatg atttttggga gaatatccaa 480
aatcatacag atgttcagcg tgtcattgat gattatgaaa ctcaggtgac gacgacactt 540
ctggatgcag gttttaagta tagtgtcata tttgacacaa ccaaagaaga tgcttcacat 600
atgctgcatg cagatttttc ttattataat ccaacagcta ttttgaacca tagagtgcct 660
tttatcaagg ttaaagctat tgataataat cagcatatta ccccctacct tttaaatgat 720
attcaaaatc attcgaccta tcctattgat ttaatcgttt ctcacatgtc agaaatcaat 780
tatcctgatt ttagttactt gttgggtcat aaatatgtca agagaaaaga agcggttgat 840
ttaacgggtc aaaaaattgc agttcatctc catgtttttt atgtggatct gctagaagaa 900
tttttgacag cattcaagca atttcatttt tcttatgatt tatttatgac aacagatagt 960
gatgataaga aagctgaaat tgaagaaatt ctagcagcaa ataatcaaga agttcaggtt 1020
tttgtcacag ggaatattgg acgtgatgtt ctccctatgt taaaattaaa aaattactta 1080
tctgcctatg attttgttgg ccattttcat accaaaaaat caaaagaagc tgatttttgg 1140
gctggccaat cttggcggga agaattaatt gacatgttgg ttaagccagc agacaatatt 1200
ttagcagaat tacagcaaaa cccgaaaatt ggtttggtta ttgctgatat gccaactttc 1260
tttcgctata ataaaattgt tgatgcttgg aatgaacatt tgattgcacc tgagatgaat 1320
acactatggc aagagatggg aatgaccaaa acgattgatt tcaatgcttt tcatactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 5
<211> 1752
<212> DNA
<213> Streptococcus mutans

<400> 5
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacta ataatcgtgc gactaagcag tttaaggaac atattcaaag ctactttatt 420
tcctttaaaa aagctgttat tcaatcggag gcctttcata atttttggga gaacatccaa 480
aatcatgcag atattcaacg tgtcattgat gattacgaaa ctcaggtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcacatgtc agaaatcaat 780
tatcctgatt ttagttattt attgggtcac aaatatgtca agaaaagaga aagagttgat 840
ttaaagaatc aaaaagttgc ggttcatctc catgtgtttt atgtggattt actggaagaa 900
tttttaacgg catttaagca atttcatttt tcttatgatt tatttataac gacagatagt 960
gatgataaga aagctgaaat tgaagagatt ctatctgcaa acagtcaaga agctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa cccaaaaatt ggtttggtga ttgctgatat gccaactttc 1260
tttcgctata ataaaattgt tgatgcttgg aatgaacatt tgattgcacc tgagatgaat 1320
acattatggc aaaagatggg catgaccaaa aagattgatt tcaatgcttt tcatactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500

gctattgagc gtttgctgat ctacattgct tggaatgagc attacgattt tagaatttct 1560
aaaaatccag ttgatctgac gcctttcata gataataaat tattaaatga acgtggcaac 1620
tcagcaccaa atacctttgt tgattttaac tatatgggag gaataaaggg agcttttaaa 1680
tatattttca ttggtccagc tagggctgtc aaatatatcc ttaaacgttc tctgcaaaaa 1740
ataaagtcat ga 1752

<210> 6
<211> 1752
<212> DNA
<213> Streptococcus mutans

<400> 6
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacca acaaccgtgc aaccaagtca tttcgtgagc atattcaaag ttactttatt 420
tcatttaaag catctgtttt aagaagcacc gctttcagag acttttggga aaatataaaa 480
gagtatcagg atgttcaaaa ggtgattgat cagtatgaaa caaaggtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcacatgtc agaaatcaat 780
tatcctgatt ttagttattt attgggtcac aaatatgtca agaaaagaga aagagttgat 840
ttaaagaatc aaaaagctgc ggttcatctc catgtgtttt atgtggattt gctggaagaa 900
tttttaacgg catttaagca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa ccccaaaatt ggtttggtga ttgctgatat gccaactttc 1260
tttcgctata ataaaattgt tgatgcttgg aatgaacatt tgattgcacc tgagatgaat 1320
acattatggc aaaagatggg catgaccaaa aagattgatt tcaatgcttt tcacactttt 1380
gtcatgagtt atggcacttt tgtttggttt aaatatgatg ccttaaaacc gctctttgat 1440
ttaaatctga cagatgatga tgtgcctgag gaacctttgc cgcaaaattc tattttacat 1500
gctattgagc gtttattgat ctacattgct tggaatgagc attacgattt tagaatttct 1560
aaaaatccag ttgatctgac gcctttcata gataataaat tattaaatga acgtggcaac 1620
tcagcaccaa atacctttgt tgattttaac tatatgggag gaataaaggg agcttttaaa 1680
tatattttca ttggtccagc tagggctgtc aaatatatcc tgaagcgttc tctgcaaaaa 1740
ataaagtcat ga 1752

<210> 7
<211> 1742
<212> DNA
<213> Streptococcus mutans

<400> 7
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacca acaaccgtgc gaccaagtca tttcgtgagc atattcaaag ttactttatt 420
tcatttaaag catctgtttt aagaagcacc gctttcagag acttttggga aaatataaaa 480
gagtatcagg atgttcaaaa ggtgattgac cagtatgaaa caaaagtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcatatgtc agaaatcaat 780
tatcctgatt ttagttattt attgggtcac aaatatgtca agaaaagaga aagagttgat 840
ttaaagaatc aaaaagttgc ggttcatctc catgtgtttt atgtggattt actggaagaa 900
tttttaacgg catttaagca atttcatttt tcttatgatt tatttataac gacagatagt 960
gatgataaga aagctgaaat tgaagagatt ctatccgcaa acggtcaaga agctcaggtt 1020

tttgtcacag gcaatattgg acgtgatgtt cttcctatgt taaaattaaa aaattattta 1080
tctgcctatg attttgttgg tcattttcat accaaaaagt caaaggaggc tgatttttgg 1140
gctggccaat cttggcggga agaattaatt gatatgttgg ttaaaccagc agacaatatt 1200
tacagcaaaa cccaaaaatt ggtttggtga ttgctgatat gccaactttc tttcgctata 1260
ataaaattgt tgatgcttgg aatgaacatt tgattgcacc tgagatgaat acattatggc 1320
aaaagatggg catgaccaaa aagattgatt tcaatgcttt tcatactttt gtcatgagtt 1380
atggtacttt tgtttggttt aaatatgatg ccttaaaacc gctctttgat ttaaatctga 1440
cagatgatga tgtgcctgag gaacctttac cgcaaaattc tattttacat gctattgagc 1500
gtttgctgat ctacattgct tggaatgagc attacgattt tagaatttct aaaaatccag 1560
ttgatctgac gcctttcata gataataaat tattaaatga acgtggtaac tcagcaccaa 1620
atacctttgt tgattttaac tatatgggag gaataaaagg agcttttaag tatatcttta 1680
ttggtccagc tagggctgtc aaatatatcc ttaaacgttc tctgcaaaaa ataaagtcat 1740
ga 1742

<210> 8
<211> 22
<212> DNA
<213> Artificial Sequence

<220>
<223> Description of Artificial Sequence: primer

<400> 8
attcccgccg ttggaccatt cc 22

<210> 9
<211> 22
<212> DNA
<213> Artificial Sequence

<220>
<223> Description of Artificial Sequence: primer

<400> 9
ccaatgtgat tcatcccatc ac 22

<210> 10
<211> 22
<212> DNA
<213> Artificial Sequence

<220>
<223> Description of Artificial Sequence: primer

<400> 10
ccgacaaaga ccattccatc tc 22

SEQUENCE LISTING

<110> SUNTORY LIMITED

<120> NOVEL SEROTYPE STREPTOCOCCUS MUTANS AND UTILIZATION OF THE SAME

<130> SU0401

<140> PCT/JP2004/019631
<141> 2004-12-28

<150> US 60/533,076
<151> 2003-12-30

<150> JP 2004-106825
<151> 2004-03-31

<160> 10

<170> PatentIn Ver. 2.1

<210> 1
<211> 1752
<212> DNA
<213> Streptococcus mutans

<400> 1
atgaaaagac tacttttgta tgtgcatttt aataaatata atcgtgtgag ttctcatgtt 60
tactaccaac taacacaaat gcgcccctta ttttcaagag tagttttcat cacaaatagt 120
cacctatctc aggagaacca agataagctg cgcagtcaaa agttgatgga tgattttcta 180
cagagggaaa atatcggttt tgattttgca gcttggcgtg atgggatgaa tcacattggt 240
tttgacaatc ttgattctta tgatattgtc actgttatga acgatacttg ctttggacct 300
ttgtgggatg ttaaggagca ttacctgtcc tatgaaaagc aggacgagat tgatttttgg 360
ggattgacta ataatcgtgc gactaagcag tttaaggaac atattcaaag ctactttatt 420
acctttaaaa aagctgttat tcaatcggag gcctttcata atttttggga gaacatccaa 480
aatcatgcag atattcaacg tgtcattgat gattacgaaa ctcaggtgac gacaacgctt 540
ttggatgctg gttttcaata tgatgtcgtt tttgatacga ccaaagaaga tgcttcgcgt 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ctaatcgttt cgcacatgtc agaaatcaat 780
tatcctgatt ttagttattt attgggtcac aaatatgtca agaaaaggga aagagttgat 840
ttaaagaatc aaaaagctgc ggttcatctc catgtgtttt atgtggattt gctggaagaa 900
tttttaacgg catttaaaca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa ccccaaaatt ggtttggtga ttgctgatat gccaactttc 1260
tttcgctata ataaaattgt ggatgcttgg aatgaacatt tgattgcacc tgagatgaat 1320
acattatggc aaaagatggg catgaccaaa aagattgatt tcaatgcttt tcacactttt 1380

gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 2
<211> 1752
<212> DNA
<213> Streptococcus mutans

<400> 2
atgaaaagac tacttttgta tgtgcatttt aataaatata atcgtgtgag ttctcatgtt 60
tactaccaac taacacaaat gcgcccctta ttttcaagag tagttttcat cacaaatagt 120
cacctatctc aggagaacca agataagctg cgcagtcaaa agttgatgga tgattttcta 180
cagagggaaa atatcggttt tgattttgca gcttggcgtg atgggatgaa tcacattggt 240
tttgacaatc ttgattctta tgatattgtc actgttatga acgatacttg ttttggacct 300
ttgtgggatg ttaaggagca ttacctgtcc tatgaaaagc aggacgagat tgatttttgg 360
ggattgacta ataatcgtgc gactaagcag tttaaggaac atattcaaag ctactttatt 420
acctttaaaa aagctgttat tcaatcggag gcctttcata atttttggga gaacatccaa 480
aatcatgcag atattcaacg tgtcattgat gattacgaaa ctcaggtgac gacaacgctt 540
ttggatgctg gttttcaata tgatgtcgtt tttgatacga ccaaagaaga tgcttcgcat 600
atgcttcatg ccgacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaatcgttt cgcacatgtc agaaatcaat 780
tatcctgatt ttagttattt attgggtcac aaatatgtca agaaaaggga aagagttgat 840
ttaaagaatc aaaaagctgc ggttcatctc catgtgtttt atgtggattt gctggaagaa 900
tttttaacgg catttaaaca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa cccaaaaatt ggtttggtga ttgctgatat gccaactttc 1260
tttcgctata ataaaattgt ggatgcttgg aatgaacatt tgattgcacc tgagatgaat 1320
acattatggc aaaagatggg catgaccaaa aagattgatt tcaatgcttt tcacactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 3
<211> 1752
<212> DNA

<213> Streptococcus mutans

<400> 3
atgaaaagac tacttttgta tgtgcatttt aataaatata atcgtgtgag ttctcatgtt 60
tactaccaac taacacaaat gcgcccctta ttttcaagag tagttttcat cacaaatagt 120
cacctatctc aggagaacca agataagctg cgcagtcaaa agttgatgga tgattttcta 180
cagagggaaa atatcggttt tgattttgca gcttggcgtg atgggatgaa tcacattggt 240
tttgacaatc ttgattctta tgatattgtc actgttatga acgatacttg ctttggacct 300
ttgtgggatg ttaaggagca ttacctgtcc tatgaaaagc aggacgagat tgatttttgg 360
ggattgacta ataatcgtgc gactaagcag tttaaggaac atattcaaag ctactttatt 420
acctttaaaa aagctgttat tcaatcggag gcctttcata atttttggga gaacatccaa 480
aatcatgcag atattcaacg tgtcattgat gattacgaaa ctcaggtgac gacaacgctt 540
ttggatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct ttcaaatgat 720
attcaaaaga attcgaccta tcctattgat ttaatcgttt cgcacatgtc agaaatcaat 780
tatcctgatt ttagttattt attgggtcac aaatatgtca agaaaaggga aagagttgat 840
ttaaagaatc aaaaagctgc ggttcatctc catgtgtttt atgtggattt gctggaagaa 900
tttttaacgg catttaaaca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gatatgttgg ttaaaccagc agacaatatt 1200
ttagcgcaat tacagcaaaa cccaaaaatt ggtttggtga ttgctgatat gccaactttc 1260
tttcgctata ataaaattgt ggatgcttgg aatgaacatt tgattgcacc tgagatgaat 1320
acattatggc aaaagatggg catgaccaaa aagattgatt tcaatgcttt tcacactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 4
<211> 1752
<212> DNA
<213> Streptococcus mutans

<400> 4
atgaaaagac tgcttttgta tgtgcatttt aataaatata atcgtgtgag ttcccatgtt 60
tactaccaac tgacacaaat gcgcccctta ttttcaagag tagttttcat cacaaatagc 120
catctagctc aggaggacca agacaagctg cgcaatcaaa atttgatgga tgattttcta 180
cagagagaaa atatcggttt tgattttgcg gcttggcgtg atgggatgaa tcacattggc 240
tttgacaatc ttgattctta tgatattgtc actgttatga acgatacttg ctttggacct 300
ttgtgggatg ttaaggatta ttacctgtcc tatgaaaagc aagatgaagt tgatttttgg 360
ggattgacca ataatcgtgc gactaagcag tttaaggagc atattcaaag ctactttatt 420
acttttaaaa aggctgttat tcaatcaaat gcctttcatg atttttggga gaatatccaa 480
aatcatacag atgttcagcg tgtcattgat gattatgaaa ctcaggtgac gacgacactt 540

ctggatgcag gttttaagta tagtgtcata tttgacacaa ccaaagaaga tgcttcacat 600
atgctgcatg cagatttttc ttattataat ccaacagcta ttttgaacca tagagtgcct 660
tttatcaagg ttaaagctat tgataataat cagcatatta ccccctacct tttaaatgat 720
attcaaaatc attcgaccta tcctattgat ttaatcgttt ctcacatgtc agaaatcaat 780
tatcctgatt ttagttactt gttgggtcat aaatatgtca agagaaaaga agcggttgat 840
ttaacgggtc aaaaaattgc agttcatctc catgtttttt atgtggatct gctagaagaa 900
tttttgacag cattcaagca atttcatttt tcttatgatt tatttatgac aacagatagt 960
gatgataaga aagctgaaat tgaagaaatt ctagcagcaa ataatcaaga agttcaggtt 1020
tttgtcacag ggaatattgg acgtgatgtt ctccctatgt taaaattaaa aaattactta 1080
tctgcctatg attttgttgg ccattttcat accaaaaaat caaaagaagc tgatttttgg 1140
gctggccaat cttggcggga agaattaatt gacatgttgg ttaagccagc agacaatatt 1200
ttagcagaat tacagcaaaa cccgaaaatt ggtttggtta ttgctgatat gccaactttc 1260
tttcgctata ataaaattgt tgatgcttgg aatgaacatt tgattgcacc tgagatgaat 1320
acactatggc aagagatggg aatgaccaaa acgattgatt tcaatgcttt tcatactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaataa acgtggtaac 1620
tcagcaccaa atacctttat tgattttaac catatgggag gaataaaagg agcttttaag 1680
tatatcttta ttggtccagc tagggctgtc aaatatatcc ttaaacgttc tctgcaaaaa 1740
ataaagtcat ga 1752

<210> 5
<211> 1752
<212> DNA
<213> Streptococcus mutans

<400> 5
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacta ataatcgtgc gactaagcag tttaaggaac atattcaaag ctactttatt 420
tcctttaaaa aagctgttat tcaatcggag gcctttcata atttttggga gaacatccaa 480
aatcatgcag atattcaacg tgtcattgat gattacgaaa ctcaggtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcacatgtc agaaatcaat 780
tatcctgatt ttagttattt attgggtcac aaatatgtca agaaaagaga aagagttgat 840
ttaaagaatc aaaaagttgc ggttcatctc catgtgtttt atgtggattt actggaagaa 900
tttttaacgg catttaagca atttcatttt tcttatgatt tatttataac gacagatagt 960
gatgataaga aagctgaaat tgaagagatt ctatctgcaa acagtcaaga agctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa cccaaaaatt ggtttggtga ttgctgatat gccaactttc 1260

tttcgctata ataaaattgt tgatgcttgg aatgaacatt tgattgcacc tgagatgaat 1320
acattatggc aaaagatggg catgaccaaa aagattgatt tcaatgcttt tcatactttt 1380
gtcatgagtt atggcacttt tgtttggttt aaatatgatg ccttaaaacc gctctttgat 1440
ttaaatctga cagatgatga tgtgcctgag gaacctttac cgcaaaattc tattttacat 1500
gctattgagc gtttgctgat ctacattgct tggaatgagc attacgattt tagaatttct 1560
aaaaatccag ttgatctgac gcctttcata gataataaat tattaaatga acgtggcaac 1620
tcagcaccaa atacctttgt tgattttaac tatatgggag gaataaaggg agcttttaaa 1680
tatattttca ttggtccagc tagggctgtc aaatatatcc ttaaacgttc tctgcaaaaa 1740
ataaagtcat ga 1752

<210> 6
<211> 1752
<212> DNA
<213> Streptococcus mutans

<400> 6
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacca acaaccgtgc aaccaagtca tttcgtgagc atattcaaag ttactttatt 420
tcatttaaag catctgtttt aagaagcacc gctttcagag acttttggga aaatataaaa 480
gagtatcagg atgttcaaaa ggtgattgat cagtatgaaa caaaggtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcacatgtc agaaatcaat 780
tatcctgatt ttagttattt attgggtcac aaatatgtca agaaaagaga aagagttgat 840
ttaaagaatc aaaaagctgc ggttcatctc catgtgtttt atgtggattt gctggaagaa 900
tttttaacgg catttaagca atttcatttt tcttatgatt tatttataac gacagatagt 960
gatgataaga aagctgaaat tgaagagatt ctatctgcaa acagtcaaga ggctcaggtt 1020
tttgtcacag gcaatattgg acgtgatgtt cttcctatgt taaaattaaa aaattattta 1080
tctacctatg attttgttgg tcattttcat accaaaaagt caaaggaggc tgatttttgg 1140
gctggccaat cttggcggga agaattaatt gacatgttgg ttaaaccagc agacaatatt 1200
ttagcgcaat tacagcaaaa ccccaaaatt ggtttggtga ttgctgatat gccaactttc 1260
tttcgctata ataaaattgt tgatgcttgg aatgaacatt tgattgcacc tgagatgaat 1320
acattatggc aaaagatggg catgaccaaa aagattgatt tcaatgcttt tcacactttt 1380
gtcatgagtt atggcacttt tgtttggttt aaatatgatg ccttaaaacc gctctttgat 1440
ttaaatctga cagatgatga tgtgcctgag gaacctttgc cgcaaaattc tattttacat 1500
gctattgagc gtttattgat ctacattgct tggaatgagc attacgattt tagaatttct 1560
aaaaatccag ttgatctgac gcctttcata gataataaat tattaaatga acgtggcaac 1620
tcagcaccaa atacctttgt tgattttaac tatatgggag gaataaaggg agcttttaaa 1680
tatattttca ttggtccagc tagggctgtc aaatatatcc tgaagcgttc tctgcaaaaa 1740
ataaagtcat ga 1752

<210> 7

<211> 1742
<212> DNA
<213> Streptococcus mutans

<400> 7
atgaagcgcc tgcttttata tgttcatttt aataaataca atcgggtaag ttcccatgtc 60
gtttatcagt tgactcaaat gagatccttg ttttcaaaag ttatctttat ttcaaatagc 120
caagtggcag atgcggatgt caaaatgcta agagaaaagc atctcattga tgacttcatt 180
caacggcaga attctggatt tgactttgca gcttggcgag atggaatggt ctttgtcggt 240
tttgatgaac ttgtgacata tgactcggta acaaccatga atgacacttg ttttggacct 300
ctttgggaaa tgtattcaat ttatcaagaa tttgaaacca agacgacagt tgatttttgg 360
ggattgacca acaaccgtgc gaccaagtca tttcgtgagc atattcaaag ttactttatt 420
tcatttaaag catctgtttt aagaagcacc gctttcagag acttttggga aaatataaaa 480
gagtatcagg atgttcaaaa ggtgattgac cagtatgaaa caaaagtcac gacaactctc 540
ttagatgctg gttttcaata tgatgtcgtt tttgatacga ccaaggaaga tgcttcgcat 600
atgcttcatg cagacttctc ttactataat ccaacagcta ttttgaatca tagggtgccc 660
tttatcaagg ttaaagcgat tgacaataat caacatatta cgccctatct tttaaatgat 720
attcaaaaga attcgaccta tcctattgat ttaattgttt cgcatatgtc agaaatcaat 780
tatcctgatt ttagttattt attgggtcac aaatatgtca agaaaagaga aagagttgat 840
ttaaagaatc aaaaagttgc ggttcatctc catgtgtttt atgtggattt actggaagaa 900
tttttaacgg catttaagca atttcatttt tcttatgatt tatttataac gacagatagt 960
gatgataaga aagctgaaat tgaagagatt ctatccgcaa acggtcaaga agctcaggtt 1020
tttgtcacag gcaatattgg acgtgatgtt cttcctatgt taaaattaaa aaattattta 1080
tctgcctatg attttgttgg tcattttcat accaaaaagt caaaggaggc tgatttttgg 1140
gctggccaat cttggcggga agaattaatt gatatgttgg ttaaaccagc agacaatatt 1200
tacagcaaaa cccaaaaatt ggtttggtga ttgctgatat gccaactttc tttcgctata 1260
ataaaattgt tgatgcttgg aatgaacatt tgattgcacc tgagatgaat acattatggc 1320
aaaagatggg catgaccaaa aagattgatt tcaatgcttt tcatactttt gtcatgagtt 1380
atggtacttt tgtttggttt aaatatgatg ccttaaaacc gctctttgat ttaaatctga 1440
cagatgatga tgtgcctgag gaacctttac cgcaaaattc tattttacat gctattgagc 1500
gtttgctgat ctacattgct tggaatgagc attacgattt tagaatttct aaaaatccag 1560
ttgatctgac gcctttcata gataataaat tattaaatga acgtggtaac tcagcaccaa 1620
atacctttgt tgattttaac tatatgggag gaataaaagg agcttttaag tatatcttta 1680
ttggtccagc tagggctgtc aaatatatcc ttaaacgttc tctgcaaaaa ataaagtcat 1740
ga 1742

<210> 8
<211> 22
<212> DNA
<213> Artificial Sequence

<220>
<223> Description of Artificial Sequence: primer

<400> 8
attcccgccg ttggaccatt cc 22

<210> 9

<211> 22
<212> DNA
<213> Artificial Sequence

<220>
<223> Description of Artificial Sequence: primer

<400> 9
ccaatgtgat tcatcccatc ac                                                22

<210> 10
<211> 22
<212> DNA
<213> Artificial Sequence

<220>
<223> Description of Artificial Sequence: primer

<400> 10
ccgacaaaga ccattccatc tc                                                22